# FASCICULE DE BREVET EUROPEEN

(11) **EP 4 340 833 B1**
(45) Date de publication et mention de la délivrance du brevet: **03.09.2025**
(21) Numéro de dépôt: 22732293.0
(22) Date de dépôt: 23.05.2022
(51) Int. Cl.: A61K 31/437, A61K 31/444, A61K 45/06, A61P 31/12, A61P 31/14, A61P 31/16, A61P 31/20

(54) **NOUVEAUX DERIVES AZAINDOLE EN TANT QU'AGENTS ANTIVIRAUX**
NEUE AZAINDOLDERIVATE ALS ANTIVIRALE MITTEL
NOVEL AZAINDOLE DERIVATIVES AS ANTIVIRAL AGENTS

(30) Priorité: 21.05.2021 FR 2105360
(43) Date de publication de la demande: 27.03.2024
(73) Titulaire: Centre National de la Recherche Scientifique (CNRS), 75016 Paris (FR); Université de Montpellier, 34090 Montpellier (FR)
(72) Inventeur: BRIANT, Laurence, 30660 GALLARGUES-LE-MONTUEUX (FR); BERNARD, Eric, 34090 MONTPELLIER (FR); CLOP, Camille, 30210 Fournès (FR)
(74) Mandataire: Novagraaf Technologies
(86) Numéro de dépôt international: PCT/FR2022/050977
(87) Numéro de publication internationale: WO 2022/243648

(56) Documents cités:
- WO-A1-2005/095400
- WO-A1-2006/015123
- WO-A1-2014/085795
- WO-A2-2010/007114

## Description

### DOMAINE DE L'INVENTION

La présente invention concerne des composés dérivés de 7-azaindole utiles en tant qu'inhibiteurs des kinases AXL pour le traitement des infections virales. La présente invention concerne également leur procédé de préparation.

### ARRIERE-PLAN TECHNOLOGIQUE

AXL est un Récepteur Tyrosine Kinase (RTK) appartenant à la famille TAM composée de TYRO-3, AXL et MER. Initialement découvert et mis en évidence chez des patients atteints de leucémie myéloïde chronique (LMC), l'implication du récepteur AXL dans des infections virales comme par exemple la dengue, le virus Zika, le Chikungunya ou encore la fièvre Ebola a été décrite dans la littérature (Chen, J. et al. Nature Microbiology, 2018, 3, pp.302-309 ; Fedeli, C. et al. Journal of Virology, 2018, 92:e01613-17 ; Hastings, A.K. et al., iScience, 2019, 13 pp.339-350 ; Meertens, L. et al., Cell Reports, 2017, 18, 3, pp.324-333). Ce récepteur, en interagissant via l'adaptateur Gas6 avec les phosphatidylsérines présentes dans les enveloppes virales, favorise l'attachement des virus enveloppés à leur cible cellulaire. AXL stimule ainsi l'endocytose des virus enveloppés dans leurs cibles cellulaires. L'engagement d'AXL au cours de cette interaction, stimule la phosphorylation du domaine intracytoplasmique d'AXL et active les voies de signalisation associées. Ces signaux neutralisent la production d'interférons et les réponses antivirales associées, favorisant l'échappement de ces virus au contrôle immunitaire de l'hôte.

A ce jour, il existe plusieurs inhibiteurs de kinase actifs sur AXL (Myers et al., J. Med. Chem. (2015), 59(8): 3593-3608) mais aucun n'est réellement sélectif ou spécifique de cette kinase. Dans une grande majorité des cas, l'activité sur AXL est secondaire vis-à-vis de l'activité principale recherchée sur MET ou MER du fait de la similarité de séquence entre ces RTKs. C'est notamment le cas pour le Bosutinib ou le Cabozantinib, qui sont des inhibiteurs de kinase multi-cibles ou MTKI (*MultiTargeted Kinase Inhibitors*) déjà sur le marché, ou bien le BMS777607 (actuellement en phase clinique 2). C'est également le cas du dérivé de 7-azaindole NPS-1034, qui présente un profil d'inhibition relativement large, en inhibant un large panel de kinases comme AXL/DDR1/FLT3/KIT/MEK/MET/ROS1 et TIE1.

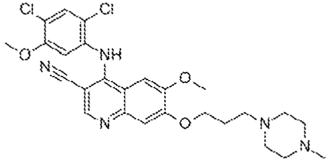

### Bosutinib

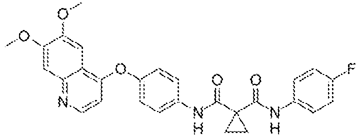

### Cabozantinib

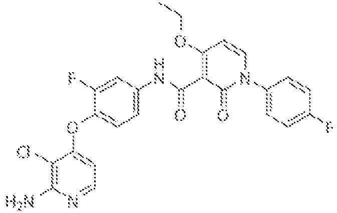

### BMS-777607

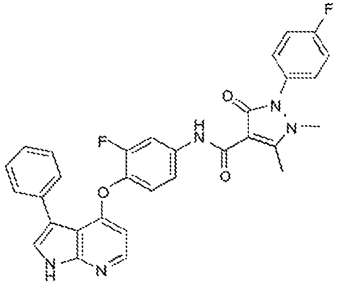

### NPS-1034

Un autre inhibiteur de la kinase AXL est le R428 (encore appelé BGB324), actuellement en phase clinique. Ce composé - de structure bien différente des inhibiteurs de kinases actuellement sur le marché - s'est également révélé actif sur d'autres kinases comme ABL/KIT/JAK2-3/LCK/PDGFRB/TIE2.

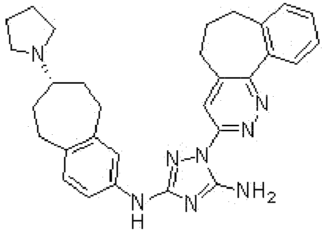

### R428/BGB324

Les documents WO 2020/007114 et WO 2006/015123 décrivent des dérivés de 7-azaindole pour leur utilisation dans le traitement des infections virales.

Il existe donc un besoin pour de nouveaux composés inhibiteurs d'AXL plus sélectifs et efficaces vis à vis des infections virales.

La présente invention propose ainsi de nouveaux dérivés de 7-azaindole inhibant fortement la kinase AXL en vue d'une utilisation comme agents antiviraux.

De par ce profil d'inhibition bien spécifique et unique à ce jour pour ce type de structure, ces composés peuvent être utilisés en thérapie dans le traitement des infections causées par des virus utilisant le récepteur AXL pour se multiplier. Afin de combattre les infections virales, en inhibant la phosphorylation d'AXL, ces composés vont agir d'une part en bloquant l'endocytose des virus enveloppés dans leur cible cellulaire, et d'autre part en neutralisant les signaux intracellulaires contrôlés par AXL qui inhibent la production des interférons et des réponses antivirales de l'hôte. Les inhibiteurs de la présente invention peuvent ainsi être utilisés pour le traitement de maladies dans lesquelles AXL sont impliquées, en particulier les infections virales et notamment l'entrée virale dans les cellules.

### RESUME DE L'INVENTION

La présente invention a ainsi pour objet un composé de formule (I): 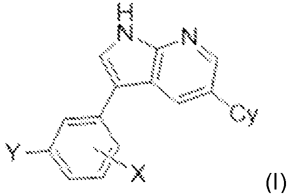 dans laquelle
X représente un atome d'hydrogène ou un atome d'halogène,
Y est choisi parmi -CH₂OH, -CH=NOH, -CH₂NH₂, un aryle, un hétéroaryle, et -L-(CH₂)p-W,
   - L représente -C(O)NH-, -CH₂NH-, -CH=N-, -NHC(O)-, ou -CH₂N=CH-,
   - p est un entier de 0 à 2,
   - quand L représente -CH₂NH-, -CH=N-, ou -CH₂N=CH-, W est choisi parmi :
      - un alkyle en C₁-C₆,
      - un aryle en C₆-C₁₀ éventuellement mono- ou polysubstitué ; et
      - un hétéroaryle à 5-10 chaînons contenant de 1 à 3 hétéroatomes indépendamment choisis parmi N, O et S, ledit hétéroaryle étant optionnellement mono- ou polysubstitué ;
   - quand L représente -C(O)NH- ou -NHC(O)-, W est choisi parmi:
      - un aryle en C₆-C₁₀ optionnellement mono- ou polysubstitué ;
      - un hétéroaryle à 5-10 chaînons comprenant de 1 à 2 hétéroatomes indépendamment choisis parmi N, O et S, ledit groupe hétéroaryle étant optionnellement mono- ou polysubstitué; et
      - un cycloakyle en C₃-C₆ optionnellement substitué par un groupe C(O)NHR ou NHC(O)R dans lequel R représente un aryle en C₆-C₁₀ éventuellement substitué par un atome d'halogène et/ou un alkyle en C₁-C₆, notamment le groupe 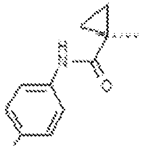
Cy représente un groupe phényle ou un groupe hétéroaryle à 5-10 chaînons contenant de 1 à 3 hétéroatomes indépendamment choisis parmi N, O ou S, ledit hétéroaryle étant optionnellement substitué par un groupe oxo, un sel pharmaceutiquement acceptable de celui-ci ou un de leurs mélanges, pour son utilisation dans la prévention et/ou le traitement des infections virales associées au récepteur AXL comme définies dans les revendications.

La présente invention a également pour objet une composition pharmaceutique comprenant un composé selon l'invention ou un sel pharmaceutiquement acceptable de celui-ci en tant que principe actif, et un excipient pharmaceutiquement acceptable, pour son utilisation dans la prévention et/ou le traitement des infections virales associées au récepteur AXL comme définies dans les revendications.

### DESCRIPTION DETAILLEE

En règle générale, les termes et définitions suivantes sont utilisés.

L'expression « couplage peptidique » dans la présente invention désigne la réaction permettant de former une liaison amide -NH-C(O)-. Les techniques utilisées dans cette réaction sont communes aux synthèses peptidiques, c'est-à-dire procèdent par activation d'un acide carboxylique pour réagir avec une amine. Les réactions de couplage peptidique utilisées dans la présente invention sont ainsi dérivées des synthèses peptidiques, et directement applicables à l'objet de la présente invention.

Les réactions de couplage peptidique sont bien connues de l'homme du métier, et peuvent notamment être réalisées en employant un agent de couplage tel que, le N,N'-dicyclohexylcarbodiimide (DCC) ou le chlorhydrate du 1-éthyl-3-(3'-diméthylaminopropyl)carbodiimide (EDC), ou le N-hydroxy-5-norbornene-2,3-dicarbodiimide), ou un benzotriazole (tel le tétrafluoroborate du O-(1H-benzotriazol-1-yl)-N,N,N',N'-tétraméthyluronium (TBTU), hexafluorophosphate de benzotriazol-1-yl-oxytris(diméthylamino)phosphonium (BOP), hexafluorophosphate du O-(7-azabenzotriazol-1-yl)-1,2,3-tétraméthyluronium (HATU), hexafluorophosphate du O-benzotriazol-1-yl-N,N,N',N'-tétraméthyluronium (HBTU), tétrafluoroborate du O-benzotriazol-1-yl-tétraméthyle (TBTU)), ou un mélange N-hydroxybenzotriazole (HOBT)/EDCI, dans un solvant tel que le chloroforme, le dichlorométhane, le dichloroéthane, l'acétate d'éthyle, le diméthylformamide (DMF), le tétrahydrofurane (THF), le diméthylsulfoxide (DMSO), la N-méthyl pyrrolidinone (NMP), de préférence à température comprise entre 20°C et 150°C.

Alternativement, un couplage peptidique a lieu par activation dans un premier temps de l'acide carboxylique par transformation en le chlorure d'acyle (notamment en présence de chlorure de thionyle ou de chlorure d'acétyle) ou un anhydride correspondant (par exemple en présence d'anhydride acétique ou isopropylique), puis réaction avec l'amine souhaitée, de préférence en présence d'une base pour neutraliser l'acide libéré lors de la réaction (notamment HCl dans le cas d'un chlorure d'acyle).

Le terme C(O) est équivalent à « C=O ».

L'expression « groupe alkyle » ou « alkyle » dans la présente invention désigne un groupe aliphatique saturé linéaire ou ramifié contenant 1 à 6 atomes de carbone, si cela n'est pas explicité. Des exemples de groupes alkyles couverts par l'objet de la présente invention sont les groupes méthyle, éthyle, propyle, butyle, tert-butyle, isopropyle.

Dans certains modes de réalisation, il est précisé qu'un ou plusieurs atomes d'hydrogène du groupe alkyle sont optionnellement remplacés par un atome de fluor. Dans ce cas, de préférence, 1 à 3 atomes d'hydrogène au plus sont concernés. Un exemple est le groupe CH₂CF₃.

L'expression "groupe aryle" ou « aryle » dans la présente invention désigne un groupe cyclique aromatique (mono- ou polycyclique) contenant entre 6 et 10 atomes de carbone. Des exemples de groupes aryles couverts par l'objet de la présente invention sont les groupes phényles, napthyles, de préférence phényle.

L'expression "groupe hétéroaryle" ou « hétéroaryle » dans la présente invention désigne un groupe cyclique aromatique (mono- ou polycyclique) à 5 à 10 chaînons, contenant entre 2 et 9 atomes de carbone et entre 1 et 3 hétéroatomes indépendamment choisis parmi l'azote, l'oxygène ou le soufre. Des exemples de groupes hétéroaryles sont les groupes furane, pyrrole, thiophène, thiazole, isothiazole, imidazole, oxazole, isoxazole, pyrazole, pyridine, pyrazine, pyridazine, pyrimidine, quinoline, indole, quinoxaline, benzofurane, dihydrobenzofurane, benzodioxole, benzotriazole, benzimidazole, de préférence choisi parmi pyrrole, imidazole, thiophène, pyridine, pyrimidine, benzofurane, benzodioxole, indole et benzimidazole.

L'expression "cycloalkyle" ou « groupe cycloalkyle » désigne un groupe aliphatique saturé cyclique contenant 3 à 6 atomes de carbone, si cela n'est pas explicité. Des exemples de groupes cycloalkyles couverts par l'objet de la présente invention sont les groupes cyclopropyle, cyclobutyle, cyclopentyle ou cyclohexyle. De préférence, il s'agit du cyclopropyle.

L'expression "atome d'halogène" dans la présente invention désigne un atome de fluor, de chlore, de brome ou d'iode. De préférence, il s'agit du brome ou du fluor, notamment du fluor. L'expression "groupe alcoxyle" ou « alcoxyle » dans la présente invention désigne un groupe alkyle lié à un oxygène. Des exemples de groupes alcoxyle sont les groupes méthoxy, éthoxy, propoxy, isopropoxy, butoxy, isobutoxy, tert-butoxy. De préférence, il s'agit d'un groupe méthoxy ou éthoxy.

L'expression "groupe aryloxy" dans la présente invention désigne un groupe aryle lié à un atome d'oxygène. Des exemples de groupes aryloxy sont les groupes phényloxy.

L'expression « groupe hydroxyle » ou « hydroxyle » dans la présente invention désigne : OH. L'expression « groupe oxo » désigne le substituant : =O.

L'expression «groupe aryle ou hétéroaryle éventuellement substitué » dans la présente invention désigne un aryle ou hétéroaryle optionnellement substitué par un ou plusieurs (de préférence 1 à 4, de manière encore préférée 1 ou 2, substituants indépendamment choisis parmi : un atome d'halogène, un groupe nitro -(NO₂), un groupe cyano (CN), un alcoxyle en C₁-C₆, un aryloxy en C₅-C₁₀, un alkyle en C₁-C₆ dans lequel 1 ou plusieurs atomes d'hydrogène est optionnellement remplacé par un atome de fluor, un hétéroaryle, un hydroxyle, un groupe -CONHalkyl en C₁-C₆, un groupe -NHCOalkyl en C₁-C₆, et un groupement NR₂R₃ dans lequel R₂ et R₃ représentent indépendamment un groupe alkyle en C₁-C₆, ou un aryle en C₆-C₁₀ éventuellement substitué par un atome d'halogène et/ou un alkyle en C₁-C₆.

De préférence, les substituants sont indépendamment choisis parmi :
∘ un atome d'halogène, notamment un fluor ou un chlore,
∘ un groupe oxo,
∘ un hydroxyle,
∘ un alcoxyle en C₁-C₆, notamment un méthoxy,
∘ un alkyle en C₁-C₆, dans lequel 1 ou plusieurs atomes d'hydrogène est optionnellement remplacé par un atome de fluor, de préférence un groupe méthyle ou CH₂CF₃, et
∘ un aryle en C₆-C₁₀ éventuellement substitué par un atome d'halogène (notamment un fluor ou un chlore) et/ou un alkyle en C₁-C₆, par exemple un fluorophényl (de préférence le 4-fluorophényle) ou un méthylfluorophényle (par exemple le 4-fluoro-2-méthylphényle);

L'expression "7-azaindole" dans la présente invention désigne le motif 1H-pyrrolo[2,3-b]pyridine:

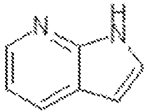

Dans la présente invention, on entend désigner par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

Dans la présente invention, l'expression « composition pharmaceutique » désigne toute composition consistant en une dose efficace d'au moins un composé de l'invention et au moins un excipient pharmaceutiquement acceptable. De tels excipients sont sélectionnés, en fonction de la forme pharmaceutique et de la méthode désirée d'administration, à partir des excipients usuellement connus par l'homme du métier.

On entend désigner par « sels pharmaceutiquement acceptables » d'un composé, des sels qui sont pharmaceutiquement acceptables, comme défini ici, et qui possèdent l'activité pharmacologique souhaitée du composé parent. De tels sels comprennent :
(1) les hydrates et les solvates,
(2) les sels d'addition d'acide pharmaceutiquement acceptable formés avec des acides inorganiques pharmaceutiquement acceptables tels que l'acide chlorhydrique, l'acide bromhydrique, l'acide sulfurique, l'acide nitrique, l'acide phosphorique et similaires ; ou formés avec des acides organiques pharmaceutiquement acceptables tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires, ou
(3) les sels d'addition de base pharmaceutiquement acceptable formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique ou inorganique pharmaceutiquement acceptable. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

L'expression « mélanges d'énantiomères » dans la présente invention désigne tout mélange d'énantiomères. Le mélange peut être racémique, c'est-à-dire 50/50 de chaque énantiomère en poids (w/w), ou non racémique, c'est-à-dire enrichi en l'un ou l'autre des énantiomères, par exemple de manière à obtenir un excès énantiomérique supérieur ou égal à 95%, de préférence supérieur ou égal à 98%, de manière encore préférée supérieur à 99%.

L'expression « mélanges de diastéréomères» dans la présente invention désigne tout mélange de diastéréomères quelle que soit la proportion.

L'expression « traitement » s'applique à tous types d'animaux, de préférence aux mammifères et plus préférentiellement aux humains. Dans le cas du traitement d'un animal non humain, l'expression référera à un traitement vétérinaire.

La présente invention concerne donc un composé de formule (I): 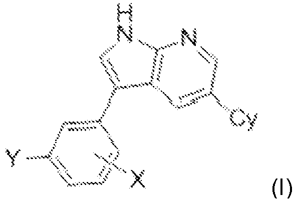 dans laquelle
X représente un atome d'hydrogène ou un atome d'halogène,
Y est choisi parmi -CH₂OH, -CH=NOH, -CH₂NH₂, un aryle, un hétéroaryle, et -L-(CH₂)p-W,
   - L représente -C(O)NH-, -CH₂NH-, -CH=N-, -NHC(O)-, ou -CH₂N=CH-,
   - p est un entier de 0 à 2,
   - quand L représente -CH₂NH-, -CH=N-, ou -CH₂N=CH-, W est choisi parmi:
      - un alkyle en C₁-C₆, de préférence un méthyle,
      - un aryle en C₆-C₁₀ , préférablement un phényle, éventuellement substitué par 1 ou 2 groupes indépendamment choisis parmi :
         ∘ un atome d'halogène,
         ∘ un hydroxyle,
         ∘ un alcoxyle en C₁-C₆, de préférence un méthoxy,
         ∘ un alkyle en C₁-C₆, dans lequel 1 ou plusieurs atomes d'hydrogène est optionnellement remplacé par un atome de fluor, de préférence un trifluorométhyle, et
         ∘ un hétéroaryle à 5-10 chaînons comprenant de 1 à 3 hétéroatomes indépendamment choisis parmi N, O et S, notamment un groupe hétéroaryle à 5 chaînons comprenant de 1 à 3 hétéroatomes indépendamment choisis parmi N, O et S tel qu'un triazole, de préférence un 1,2,4-triazole ;
      - un hétéroaryle à 5-10 chaînons contenant de 1 à 3 hétéroatomes indépendamment choisis parmi N, O et S, tel qu'un furane, pyrrole, imidazole, pyrazole, thiophene, pyridine, pyrimidine, benzofurane, benzodioxole, indole, benzimidazole, ledit hétéroaryle étant éventuellement substitué par 1 à 4 groupes indépendamment choisis parmi:
         ∘ un atome d'halogène,
         ∘ un groupe oxo
         ∘ un hydroxyle,
         ∘ un alcoxyle en C₁-C₆,
         ∘ un alkyle en C₁-C₆, dans lequel 1 ou plusieurs atomes d'hydrogène est optionnellement remplacé par un atome de fluor, et
         ∘ un aryle en C₆-C₁₀ éventuellement substitué par un atome d'halogène et/ou un alkyle en C₁-C₆, de préférence un fluorophényle (notamment le 4-fluorophényle) ou un térazole substitué par un alkyle en C₁-C₆ tel qu'un méthyle ;
   - quand L représente -C(O)NH- ou -NHC(O)-, W est choisi parmi:
      - un aryle en C₆-C₁₀ éventuellement substitué par un atome d'halogène, un hydroxyle, un alkyle en C₁-C₆, ou un alcoxyle en C₁-C₆, préférablement un atome de fluor, un hydroxyle, méthoxy ou trifluorométhyle, de manière encore préférée par un hydroxyle,
      - un hétéroaryle à 5-10 chaînons comprenant de 1 à 2 hétéroatomes indépendamment choisis parmi N, O et S, tel qu'un furane, pyrrole, imidazole, pyrazole, thiophene, pyridine, pyrimidine, benzofurane, benzodioxole, indole, benzimidazole, ledit hétéroaryle étant éventuellement substitué par 1 à 4 substituants indépendamment choisis parmi:
         ∘ un atome d'halogène,
         ∘ un hydroxyle,
         ∘ un groupe oxo,
         ∘ un alcoxyle en C₁-C₆,
         ∘ un alkyle en C₁-C₆ dans lequel 1 ou plusieurs atomes d'hydrogène est optionnellement remplacé par un atome de fluor, et
         ∘ un aryle en C₆-C₁₀ éventuellement substitué par un atome d'halogène et/ou un alkyle en C₁-C₆, de préférence un fluorophényle, notamment le 4-fluorophényle; et
      - un cycloakyle en C₃-C₆ optionnellement substitué par un groupe C(O)NHR ou NHC(O)R dans lequel R représente un aryle en C₆-C₁₀ éventuellement substitué par un atome d'halogène et/ou un alkyle en C₁-C₆, en particulier un phényle éventuellement substitué par un atome d'halogène et/ou un alkyle en C₁-C₆, de préférence un phényle éventuellement substitué par un atome d'halogène, notamment il s'agit du cyclopropyle substitué de formule 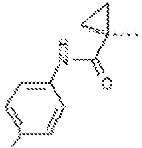
Cy représente un phényle ou un hétéroaryle à 5-10 chaînons contenant de 1 à 3 hétéroatomes indépendamment choisis parmi N, O et S, ledit hétéroaryle étant optionnellement substitué par un groupe oxo,
un sel pharmaceutiquement acceptable de celui-ci ou un de leurs mélanges,
pour son utilisation dans la prévention et/ou le traitement des infections virales associées au récepteur AXL comme définies dans les revendications.

Le composé de formule (I) selon l'invention peut se trouver sous la forme d'un stéréoisomère ou d'un mélange de stéréoisomères, tels que des tautomères, énantiomères ou diastéréoisomères.

Dans un mode de réalisation particulier de l'invention, le composé de l'invention est de formule (la): 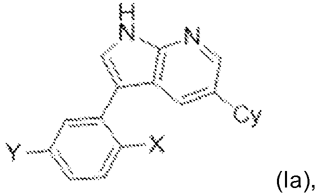 dans laquelle X, Y et Cy sont tels que définis ci-avant et ci-après.

Dans un mode de réalisation particulier, X représente un halogène, notamment le fluor. Dans un mode de réalisation particulier, X représente un hydrogène.

Cy représente de préférence un phényle, furane, pyrrole, imidazole, pyrazole, 3-oxo-2,3-dihydro-1H-pyrazole, thiophene, pyridine, 2(1H)-pyridinone, 4(1H)-pyridinone, pyrimidine, pyrimidine-2,4-dione, benzofurane, benzodioxole, indole ou benzimidazole. Dans certains modes de réalisation, Cy représente un phényle ou un hétéroaryle monocyclique à 5-7 chaînons contenant de 1 à 3 hétéroatomes indépendamment choisis parmi N, O ou S. Ainsi, de manière encore préférée, Cy représente un phényle, thiophène, furane, pyridine ou pyrimidine. De manière encore plus préférée, Cy représente un phényle, thiophène, pyridine ou pyrimidine. En particulier, Cy représente un phényle ou un thiophène.

Dans un mode de réalisation particulier, Y représente -CH=NOH, CH₂NH₂, aryle ou hétéroaryle optionnellement mono ou polysubstitué.

Dans un autre mode de réalisation particulier, Y représente -CH₂OH.

Dans un autre mode de réalisation particulier, Y représente -L-(CH₂)p-W, avec L, p et W tels que définis ci-avant ou ci-après.

Par exemple, quand L représente -CH₂NH-, -CH=N-, ou -CH₂N=CH-, de préférence -CH₂NH-, p est compris entre 0 et 2, de préférence égal à 1 ou 2, préférablement 1, et W est avantageusement choisi parmi:
- un phényle éventuellement substitué par 1 ou 2 groupes indépendamment choisis parmi un atome de brome, un atome de fluor, un hydroxyle, un méthoxy, un trifluorométhyle, et un triazole, de préférence parmi un atome de brome, un atome de fluor, un hydroxyle, un méthoxy et un 1,2,4-triazole, et
- un hétéroaryle choisi parmi un furane, pyrrole, imidazole, pyrazole, thiophene, pyridine, pyrimidine, benzofurane, benzodioxole, indole et benzimidazole, de préférence choisi parmi indole, pyridine, imidazole, benzimidazole, benzofurane, benzodioxole et pyrazole, ledit hétéroaryle étant optionnellement substitué par 1 à 4 groupes indépendamment choisis parmi: un atome d'halogène (notamment le fluor ou le brome), un groupe oxo, un hydroxyle, un méthoxy, un méthyle, un trifluorométhyle, un phényle et un fluorophényle, notamment choisi parmi un méthyle, un phényle et le 4-fluorophényle.

En particulier, dans ce mode de réalisation, W peut être choisi parmi un furane, pyrrole, imidazole, pyrazole, 3-oxo-2,3-dihydro-1H-pyrazole, thiophene, pyridine, 2(1H)-pyridinone, 4(1H)-pyridinone, pyrimidine, pyrimidine-2,4-dione, benzofurane, benzodioxole, indole, benzimidazole, optionnellement substitué par 1 à 4 groupes indépendamment choisis parmi un méthyle, un phényle et le 4-fluorophényle.

En particulier, quand L représente CH₂NH, CH=N, ou CH₂N=CH, de préférence CH₂NH, p est de préférence égal à 1 ou 2, notamment 1, et W est alors de préférence choisi parmi :

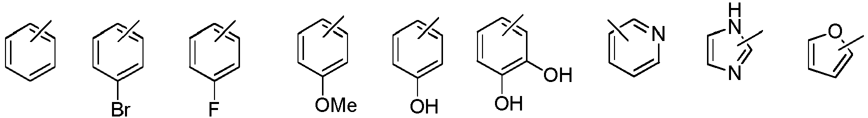

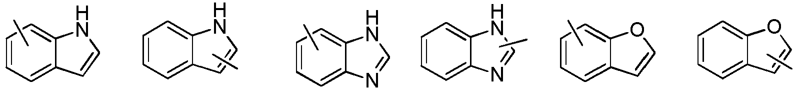

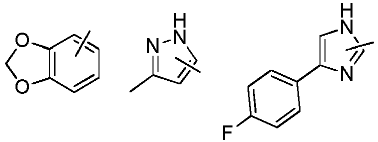

Encore plus préférablement, W est un imidazole, notamment non substitué.

Dans le mode de réalisation où L représente -CH₂NH-, -CH=N-, ou -CH₂N=CH-, de préférence -CH₂NH-, X représente de préférence un halogène, notamment le fluor, et Cy représente avantageusement un phényle ou un hétéroaryle monocyclique à 5-7 chaînons contenant de 1 à 3 hétéroatomes indépendamment choisis parmi N, O ou S, en particulier un thiophène.

En outre, quand L représente -C(O)NH- ou -NHC(O), de préférence - NHC(O)-, p est compris entre 0 et 2, de préférence égal à 0 ou 1, notamment 0, et W est avantageusement choisi parmi:
- un phényle ou naphtalène optionnellement mono- ou polysubstitué, avantageusement par un atome d'halogène, un hydroxyle, un alkyle en C₁-C₆, ou un alcoxyle en C₁-C₆, préférablement un atome de fluor, un hydroxyle, méthoxy ou trifluorométhyle, de manière encore préférée par un hydroxyle,
- un hétéroaryle choisi parmi un furane, pyrrole, imidazole, pyrazole, thiophene, pyridine, pyrimidine, benzofurane, benzodioxole, indole et benzimidazole, ledit hétéroaryle étant optionnellement substitué par 1 à 4 substituants indépendamment choisis parmi:
   ∘ un atome d'halogène, notamment le fluor,
   ∘ un hydroxyle,
   ∘ un groupe oxo,
   ∘ un méthoxy,
   ∘ un méthyle ou trifluorométhyle, et
   ∘ un aryle en C₆-C₁₀ éventuellement substitué par un atome d'halogène et/ou un alkyle en C₁-C₆, de préférence un fluorophényle, notamment le 4-fluorophényle, et
- un cycloakyle en C₃-C₆ optionnellement substitué par un groupe -C(O)NHR ou -NHC(O)R dans lequel R représente un aryle en C₆-C₁₀ éventuellement substitué par un atome d'halogène et/ou un alkyle en C₁-C₆, notamment le cyclopropyle substitué de formule

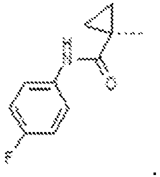

En particulier, dans ce mode de réalisation, W peut être choisi parmi un furane, pyrrole, imidazole, pyrazole, 3-oxo-2,3-dihydro-1H-pyrazole, thiophene, pyridine, 2(1H)-pyridinone, 4(1H)-pyridinone, pyrimidine, pyrimidine-2,4-dione, benzofurane, benzodioxole, indole, benzimidazole, optionnellement substitué par 1 à 4 choisis parmi:
∘ un atome d'halogène, notamment le fluor,
∘ un méthyle, et
∘ un aryle en C₆-C₁₀ éventuellement substitué par un atome d'halogène, de préférence un fluorophényle, notamment le 4-fluorophényle.

Avantageusement, quand L représente -C(O)NH- ou -NHC(O), de préférence - NHC(O)-, p est de préférence égal à 0 ou 1, notamment 0, et W est avantageusement choisi parmi:
- la 2(1H)-pyridinone, optionnellement substitué par 1 à 4 substituants, notamment 1 à 2, indépendamment choisis parmi:
   ∘ un aryle en C₆-C₁₀ éventuellement substitué par un atome d'halogène, de préférence un fluorophényle, notamment le 4-fluorophényle, et
   ∘ un méthyle, et
- un cycloakyle en C₃-C₆ optionnellement substitué par un groupe -C(O)NHR ou -NHC(O)R dans lequel R représente un aryle en C₆-C₁₀, notamment un phényle, éventuellement substitué par un atome d'halogène, notamment le cyclopropyle substitué de formule

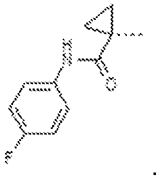

En particulier, quand L représente C(O)NH- ou -NHC(O)-, de préférence -NH(CO)-), p est de préférence égal à 0 ou 1, notamment 0 et W est alors choisi parmi : 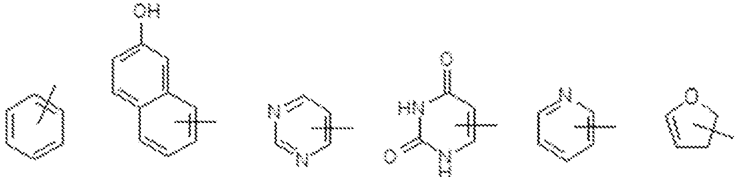 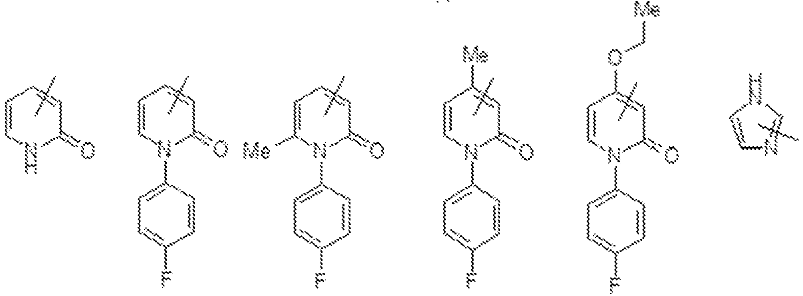 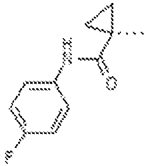 et, 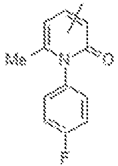 préférablement

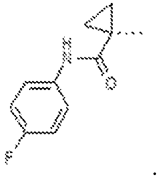

Dans le mode de réalisation où L représente -C(O)NH- ou -NHC(O), de préférence - NHC(O), X représente un atome d'hydrogène ou un atome d'halogène, notamment le fluor, et Cy représente avantageusement un phényle.

De préférence, le composé est choisi parmi : 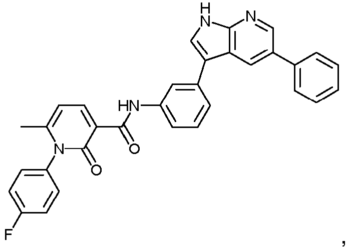 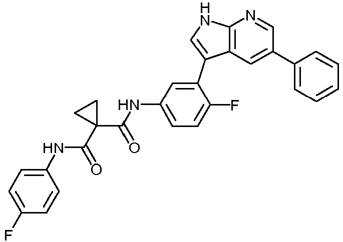 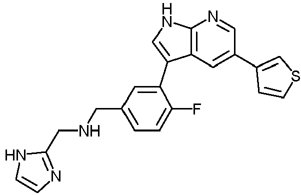 et, 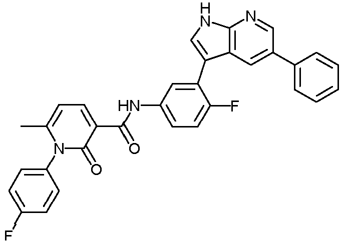 de préférence 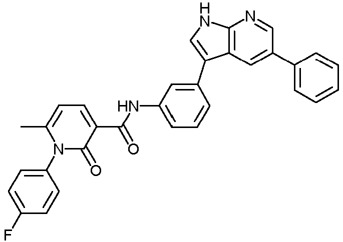 et

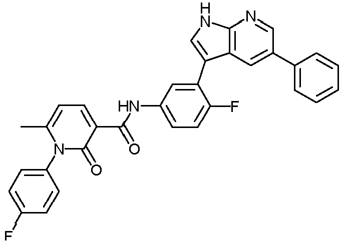

### Compositions et Applications thérapeutiques

Les composés de la présente invention inhibent les récepteurs AXL. AXL étant impliqué dans de nombreux processus biologiques et étant une cible thérapeutiquement validée, les composés de la présente invention et leurs sels pharmaceutiquement acceptables, ou les compositions de l'invention telle que définies ci-après, sont utiles en tant que médicament, notamment dans le traitement de infections virales dont le cycle infectieux dépend d'AXL et de la signalisation associée.

La présente invention porte sur des composés de formule (I) tels que définis ci-dessus pour utilisation dans la prévention et/ou le traitement des infections virales associées au récepteur AXL comme définies dans les revendications.

Plus particulièrement, les composés peuvent être employés pour préparer des compositions pharmaceutiques comprenant à titre de principe actif au moins un composé de formule (I) décrits ci-dessus ou un sel pharmaceutiquement acceptable de celui-ci, avec au moins un excipient pharmaceutiquement acceptable. Ainsi, la présente invention concerne une composition pharmaceutique comprenant un composé de formule (I) selon l'invention ou un sel pharmaceutiquement acceptable de celui-ci en tant que principe actif, et un excipient pharmaceutiquement acceptable, pour son utilisation dans la prévention et/ou le traitement des infections virales. Lesdits excipients sont choisis selon la forme pharmaceutique et le mode d'administration souhaité parmi les excipients habituels qui sont connus de l'homme du métier.

La composition pharmaceutique selon l'invention peut comprendre en outre un agent thérapeutique supplémentaire, typiquement choisi parmi un agent antiviral, un agent anti-inflammatoire, un agent immunomodulateur ou immunosuppresseur, un agent pour le traitement de troubles d'immunodéficience et un agent pour le traitement de la douleur. En particulier, il s'agit d'un agent utile dans un traitement contre les infections virales.

Un objet de l'invention concerne donc l'utilisation des composés de formule (I) tels que définis ci-dessus ou d'une composition pharmaceutique telle que définie ci-dessus dans la prévention et/ou le traitement des infections virales.

En d'autres termes, l'invention porte sur l'utilisation d'un composé de formule (I) tel que défini ci-dessus ou d'une composition pharmaceutique telle que définie ci-dessus pour la préparation d'un médicament pour la prévention et/ou le traitement des infections virales.

Les composés de formule (I) selon l'invention ou leurs sels pharmaceutiquement acceptables, ou la composition pharmaceutique selon l'invention sont particulièrement utiles pour la prévention et/ou le traitement des infections virales dues aux Flavivirus, tels que les virus de la dengue, Zika, West Nile, Kunjin, aux Alphavirus, tels que les virus Chikungunya, Mayaro, Semliki Forest, Sindbis, le virus de l'encéphalite équine de l'Est, le virus de l'encéphalite equine Occidental, le virus de l'encéphalite équine du Venezuela, aux Filovirus tels que le virus Ebola, le virus de la fièvre de Marburg, aux arénavirus tels que le virus de la fièvre de Lassa, le virus Junin, le virus Amapari, aux picornavirus tels que le virus de la stomatite vésiculeuse, aux paramyxovirus, tels que le virus influenza, ou aux coronavirus tels que le SARS-COV2.

Dans un mode de réalisation particulier, l'invention porte sur les composés de formule (I) tels que définis ci-dessus ou la composition pharmaceutique telle que définie ci-dessus dans la prévention et/ou le traitement des infections virales due à un coronavirus, notamment le SARS-Cov2.

La présente invention concerne également un kit comprenant :
a) une première composition comprenant un composé selon l'invention ou un sel pharmaceutiquement acceptable de celui-ci en tant que principe actif, et un excipient pharmaceutiquement acceptable, et
b) une deuxième composition comprenant un agent thérapeutique supplémentaire, typiquement choisi parmi un agent antiviral, un agent anti-inflammatoire, un agent immunomodulateur ou immunosuppresseur, un agent pour le traitement de troubles d'immunodéficience et d'un agent pour le traitement de la douleur, de préférence un agent utile dans un traitement contre les infections virales, en tant que produit de combinaison pour utilisation séparée, concomitante ou étalée dans le temps.

Ledit kit est utile en tant que médicament, en particulier pour la prévention et/ou le traitement des infections virales, notamment des infections virales telles que définies ci-dessus.

Les compositions pharmaceutiques selon l'invention peuvent être administrées par voie parentérale, telle que par voie intraveineuse ou intradermique, ou par voie topique, orale ou nasale.

Les formes administrables par voie parentérale incluent les suspensions aqueuses, les solutions salines isotoniques ou les solutions stériles et injectables qui peuvent contenir des agents de dispersion et/ou des mouillants pharmacologiquement compatibles. Les formes administrables par voie orale incluent les comprimés, les gélules molles ou dures, les poudres, les granules, les solutions et suspensions orales. Les formes administrables par voie nasale incluent les aérosols. Les formes administrables par voie topique incluent les patchs, les gels, les crèmes, les pommades, les lotions, les sprays, les collyres.

De préférence, les composés ou compositions de l'invention sont administrés par voie orale ou parentérale (notamment intraveineuse).

La dose efficace d'un composé de l'invention varie en fonction de nombreux paramètres tels que, par exemple, la voie d'administration choisie, le poids, l'âge, le sexe, l'état d'avancement de la pathologie à traiter et la sensibilité de l'individu à traiter.

La présente invention, selon un autre de ses aspects, concerne également un composé selon l'invention pour son utilisation dans une méthode de prévention et/ou de traitement des pathologies ci-dessus indiquées qui comprend l'administration, à un patient en ayant besoin, d'une dose efficace d'un composé selon l'invention, ou un de ses sels pharmaceutiquement acceptable ou d'une composition selon l'invention, de préférence par voie parentérale (notamment intraveineuse) ou voie orale.

### Procédé de préparation des composés de l'invention

La présente divulgation concerne également les méthodes de préparation des composés décrits ci-dessus, notamment à partir du 5-bromo-3-iodo-1H-pyrrolo[2,3-b]pyridine (II).

Selon le premier mode de réalisation, la méthode concernant la présente divulgation est représentée dans le Schéma 1. 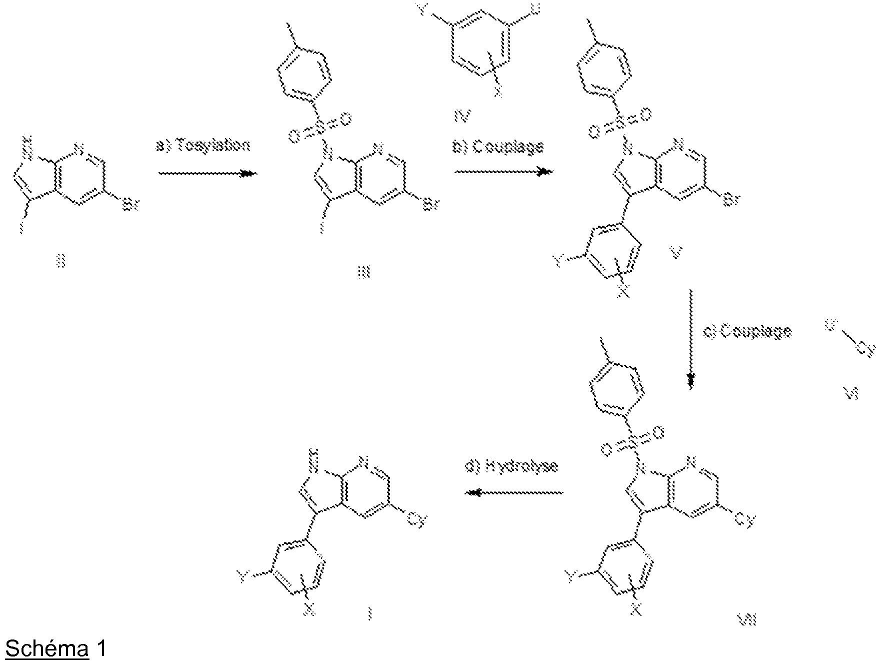 où X et Cy sont tels que définis précédemment et Y est un groupe parmi -CHO, -CN, -CH₂OH, -CO₂H ou -NO₂.

La méthode comprend au moins les étapes de :
a) tosylation du 5-bromo-3-iodo-1H-pyrrolo[2,3-b]pyridine (II), par exemple avec le chlorure de toluène-4-sulfonyle en présence d'une base comme l'hydrure de sodium, pour obtenir l'intermédiaire (III),
b) réaction de couplage de type Suzuki-Miyaura en présence d'un catalyseur au palladium comme le palladium dichloro [1,1'-Bis(diphénylphosphino)ferrocène] (Pd(dppf)Cl₂), avec l'intermédiaire de formule (IV) dans lequel U représente un acide boronique ou son ester de pinacol, pour obtenir le composé de formule (V),
c) réaction de couplage de type Suzuki-Miyaura en présence d'un catalyseur au palladium comme le palladium dichloro [1,1'-*Bis*(diphénylphosphino)ferrocène] (Pd(dppf)Cl₂), avec l'intermédiaire de formule (VI) dans lequel U' représente un acide boronique ou son ester de pinacol, pour obtenir l'intermédiaire de formule (VII), et
d) hydrolyse du groupement tosyle par une base, de préférence l'hydroxyde de sodium ou le carbonate de césium, pour obtenir un composé de formule (I).

La synthèse générale des composés intermédiaires aminés (VIII) est représentée dans le Schéma 2. 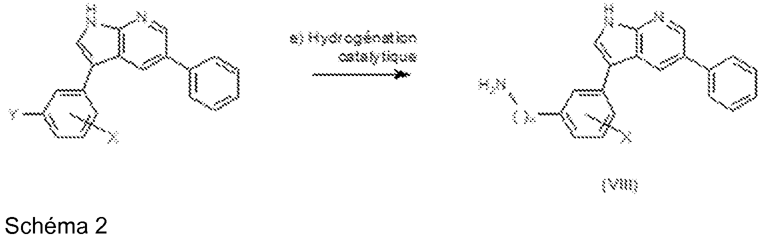 où X est tel que défini précédemment et Y est un groupement cyano ou nitro avec n égal 0 ou 1.

La méthode comprend au moins l'étape de :
e) hydrogénation catalytique des fonctions nitro ou cyano en présence de catalyseur, par exemple palladium sur charbon ou nickel de Raney, et d'hydrogène par exemple sous pression d'hydrogène ou libéré in situ par du formiate d'ammonium sous irradiation micro-ondes

L'homme du métier appliquera naturellement toutes les autres techniques de synthèse bien décrites et connues pour synthétiser ces types de composés.

Les composés de formule (I) dans lesquels Y représente un groupe L-(CH₂)ₚ-W avec L représentant un groupe amide -NHCO-, sont par exemple obtenus par une méthode de synthèse à partir des dérivés amino-7-azaindoles représentée sur le Schéma 3 : 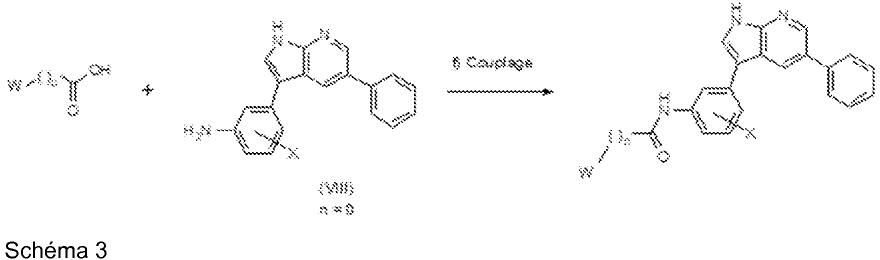 où W, X et p sont tels que définis précédemment.

La méthode du Schéma 3 comprend au moins une étape de réaction de couplage peptidique entre un acide de formule W-(CH₂)ₚ-C(O)OH et l'intermédiaire aminé de formule (VIII) tels que défini précédemment, notamment en présence d'au moins un agent activateur comme l'héxafluorophosphate de 2-(7-aza-*1H*-benzotriazol-1-yl)-*N,N,N',N'*-tétraméthyluronium (HATU) et d'une base comme la diisopropyléthylamine (DIEA).

L'homme du métier appliquera naturellement toutes les autres techniques de synthèse bien connues pour obtenir ces types de composés amides.

Les composés de formule (I) dans lesquels Y représente un groupe L-(CH₂)ₚ-W avec L représentant un groupe -CONH-, sont par exemple obtenus par une méthode de synthèse à partir des dérivés 7-azaindole carboxyliques de formule (IX) représentée sur le Schéma 4 selon deux méthodes parmi d'autres: 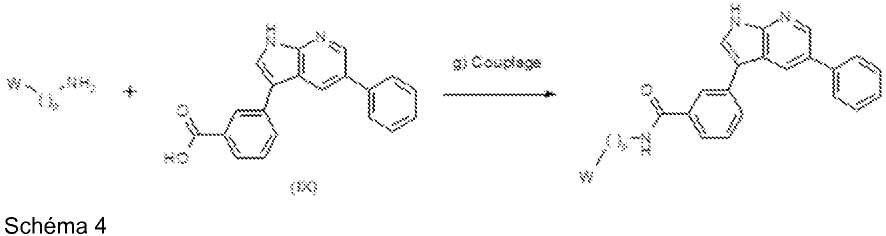 où W et p sont tels que définis précédemment.

De manière avantageuse, les méthodes du Schéma 4 comprennent au moins une étape de réaction de couplage peptidique entre un acide de formule (IX) et une amine de formule W-(CH₂)ₚ-NH₂, soit par couplage, soit par génération *in situ* des chlorures d'acyle par action préalable du chlorure de thionyle.

L'homme du métier appliquera naturellement toutes les autres techniques de synthèse bien connues pour obtenir ces types de composés amides.

Selon un autre mode de réalisation, concernant la méthode de synthèse des composés imines ou amines secondaires de la présente invention, i.e. les composés de formule (I) dans laquelle Y représente un groupe L-(CH₂)ₚ-W avec L représentant un groupe -CH=N- ou -CH₂NH-, trois méthodes parmi d'autres sont représentées dans le Schéma 5 : 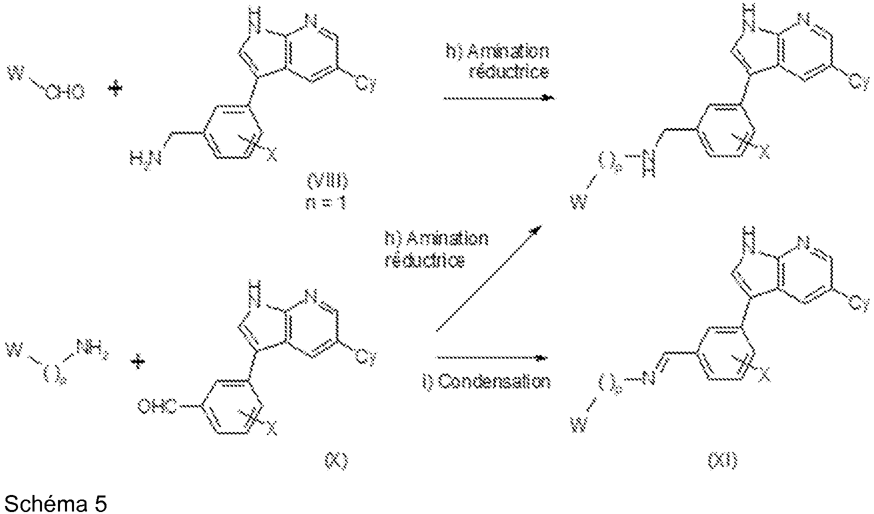 où W, X, Cy et p sont tels que définis précédemment.

De manière avantageuse, les méthodes du Schéma 5 comprennent au moins une étape d'amination réductrice entre soit, un composé de formule (VIII) dans lequel n est égal à 1, avec des aldéhydes de formule W-CHO, soit entre les 7-azaindoles aldéhydes de formule (X) et une amine de formule W-(CH₂)ₚ-NH₂.

Les méthodes du Schéma 5 comprennent également une réaction de condensation entre les composés 7-azaindoles aldéhydes de formule (X) et une amine de formule W-(CH₂)ₚ-NH₂ pour former les composés imines de formule (XI).

Les aldéhydes de formule W-CHO et les amines de formule W-(CH₂)ₚ-NH₂ sont notamment disponibles dans le commerce, ou facilement obtenus selon des procédés de préparation connus par l'homme du métier.

L'homme du métier appliquera naturellement toutes les autres techniques de synthèse bien connues pour obtenir ces types de composés imines et amines.

Une méthode pour synthétiser les composés phénols selon la présente invention est représentée dans le Schéma 6 suivant :

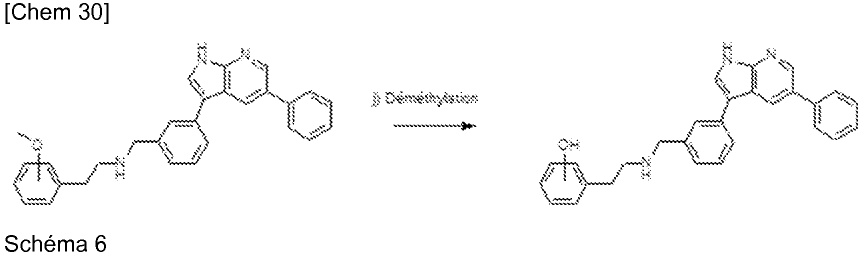

De manière avantageuse, la méthode comprend au moins l'étape suivante :
j) déméthylation du composé méthoxyphényle en présence de tribromure de bore (BBr₃) pour former l'hydroxyphényle désiré.

L'homme du métier appliquera naturellement toutes les autres techniques de synthèse bien connues pour synthétiser les hydroxyphényles souhaités.

### DESCRIPTION DES FIGURES

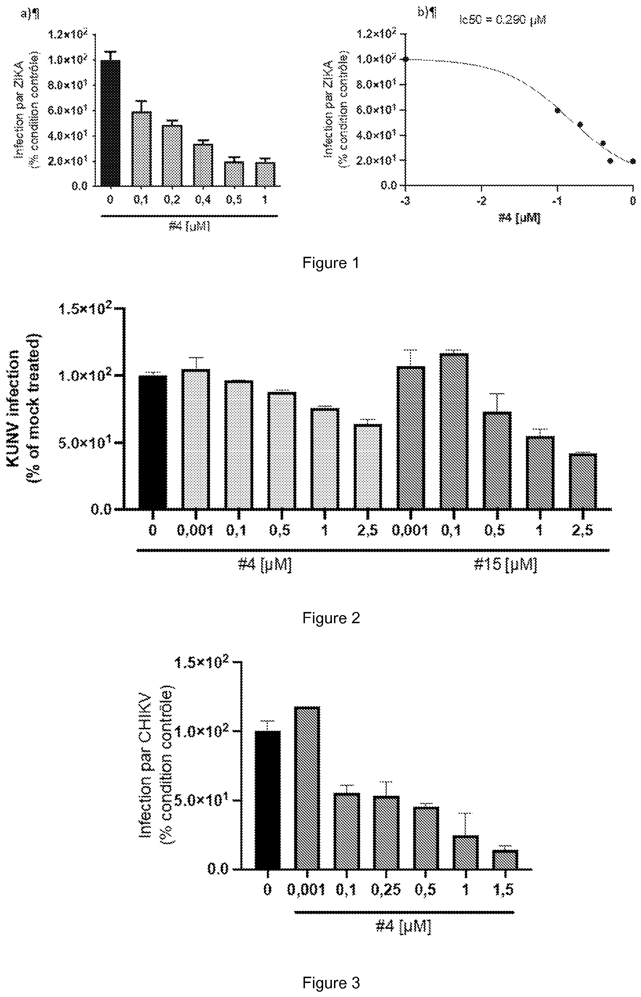

**Figure 1** : Activité anti-ZIKV du composé 4. Les cellules ont été préincubées pendant 1 heure avec les composés aux concentrations indiquées (0,1, 0,2, 0,4, 0,5, 1 µM), infectées avec la souche virale ZIKV BeH8 (MOI= 0.1), et maintenues en culture pendant 24h en présence des composés. La réplication virale résiduelle est déterminée par quantification de l'ARN génomique viral présents dans les cellules, par qRT-PCR. Les valeurs sont exprimées comme pourcentage de l'infection détectée dans la condition contrôle obtenue par incubation de cellules en présence de DMSO (0), solvant de dilution des composés. Les valeurs sont des moyennes de triplicats + écart-type : a) représentation en histogramme, b) représentation logarithmique.
**Figure 2** : Activité anti-KUNV des composés 4 et 15. Les cellules ont été préincubées pendant 1 heure avec les composés aux concentrations indiquées (0,001, 0,1, 0,5, 1 et 2,5 µM), puis infectées par un virus rapporteur KUNV-luciferase (MOI= 0.1). Les cellules sont maintenues en culture pendant 24h en présence des composés. La réplication virale résiduelle, mesurée par quantification de l'activité luciférase dans le lysat cellulaire à l'aide du réactif Genofax A(Yelen) est exprimée comme pourcentage de l'infection détectée dans la condition contrôle obtenue par incubation de cellules en présence de DMSO (0), solvant de dilution des composés. Les valeurs sont des moyennes de triplicats + écart-type.
**Figure 3** : Activité anti-CHIKV du composé 4. Les cellules ont été préincubées pendant 1 heure avec le composé aux concentrations indiquées (0,001, 0,1, 0,25, 0,5, 1 et 1,5 µM), puis infectées avec la souche virale CHIKV LR-OPY1 contenant un gène rapporteur luciferase (MOI= 0.1). Les cellules ont été maintenues en culture pendant 24h en présence du composé. La réplication virale résiduelle, mesurée par quantification de l'activité luciférase dans le lysat cellulaire (réactif Genofax Yelen), est exprimée comme pourcentage de l'infection détectée dans la condition contrôle obtenue par incubation de cellules en présence de DMSO (0), solvant de dilution du composé. Les valeurs sont des moyennes de triplicats + écart-type.

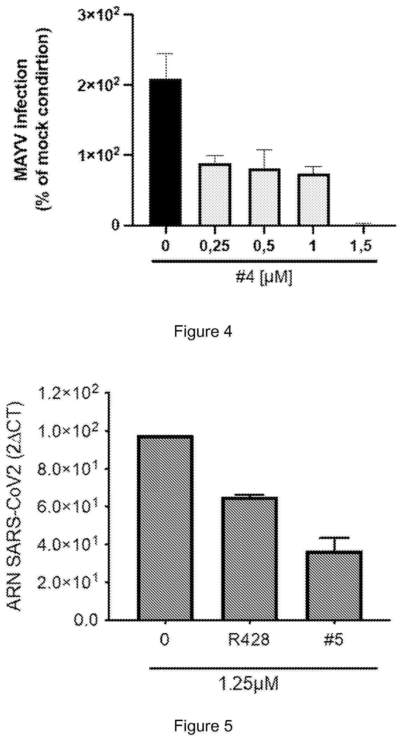

**Figure 4** **:** Activité anti-MAYV du composé 4. Les cellules ont été préincubées pendant 1 heure avec les composés aux concentrations indiquées (0,25, 0,5, 1 et 1,5 µM), puis infectées avec la souche virale TRVL4576-luc (MOI= 0.1). Les cellules ont été maintenues en culture pendant 24h en présence du composé. La réplication virale résiduelle, mesurée par quantification de l'activité luciférase dans le lysat cellulaire (réactif Genofax A, Yelen), est exprimée comme pourcentage de l'infection détectée dans la condition contrôle obtenue par incubation de cellules en présence du solvant de dilution des composés, DMSO (0). Les valeurs sont des moyennes de triplicats + écart-type.
**Figure 5** : Activité anti-SARS-CoV2 du composé 5 déterminée dans la lignée VeroE6. Les cellules ont été préincubées pendant 1 heure avec le composé 5 à une concentration de 1,25 µM, puis infectées avec la souche virale (MOI= 0.1). Les cellules ont été maintenues en culture pendant 24 heures en présence du composé. La réplication virale résiduelle est mesurée par quantification de l'ARN viral dans la culture cellulaire par qRT-PCR. La condition contrôle est obtenue par incubation de cellules en présence de DMSO (0). Le R428/Bemcentinib est évalué en parallèle. Les valeurs sont des moyennes de triplicats + écart-type.

### EXEMPLES

L'invention sera mieux comprise à la lecture des exemples suivants, qui sont donnés à titre purement illustratifs et ne devraient pas être interprétés comme limitant la portée de la présente invention.

### Exemple 1. Synthèse

### Matériel

Les synthèses et analyses ont été réalisées dans les conditions suivantes.

### Résonance Magnétique Nucléaire ¹H et ¹³C:

*Appareil* : Bruker Avance 400 (400 MHz); Bruker Avance 300 (300 MHz)
*Conditions d'utilisation* : Température ambiante, déplacements chimiques exprimés en partie par million (ppm), multiplicité des signaux indiquée par des lettres minuscules (singulet s, doublet d, triplet t, quadruplet q, multiplet m), sulphoxyde de diméthyle d₆, méthanol d₄, chloroforme d₁ comme solvants deutérés.

### Chromatographie Liquide Haute Pression (HPLC):

*Appareil* : Agilent Technology 1260 Infinity
*Conditions d'utilisation :* Colonne Zorbax SB-C18 ou Eclipse plus (2.1 x 50 mm), 1.8 µm; température: 30°C, débit : 0.5 mL/min pour les méthodes X et Y et 0.4 mL/min pour la méthode Z, gradient d'élution Eau (A) /Acétonitrile (B) /Acide formique 0.1% (Temps (min)/% B) :
   - Method X: 0/10, 0.3/10, 5.7/100, 6.0/100
   - Method Y: 0/10, 1/50, 6/100, 8/100
   - Method Z: 0/10, 11/100, 15/100

### Spectrométrie de Masse (MS) :

*Appareil*: Quadripole Agilent Technologies 6120
*Conditions d'utilisation* : ElectroSpray (ESI) en mode positif et/ou négatif.

### Pesées :

*Appareil* : Denver Instrument TP214 (précision 0.1 mg)
*Conditions d'utilisation :* Pesées effectuées au milligramme près.

### Réactions sous pression :

*Appareil :* Autoclave Parr 300 mL.
*Conditions d'utilisation* : Hydrogénation sous 20 bars d'hydrogène.

### Réaction sous irradiation micro-ondes :

*Appareil* : CEM Discover SP^{®}
*Conditions d'utilisation* : Puissance de 200 Watts, Vitesse d'agitation moyenne

Dans la suite, les abréviations suivantes sont utilisées :

| | | | |
|---|---|---|---|
| eq | équivalent | DMF | N,N-diméthylformamide |
| TA | Température ambiante | DMSO | diméthylsulfoxyde |

### Synthèse générale des intermédiaires 3,5-diaryl-1H-pyrrolo[2,3-b]pyridines (I)

### 1^{ère} étape, synthèse de la 5-bromo-3-iodo-1-(toluène-4-sulfonyl)-1H-pyrrolo[2,3-b]pyridine (III)

3 g de 5-bromo-3-iodo-1*H*-pyrrolo[2,3-b]pyridine (II) sont dilués dans 60 mL de THF anhydre sous argon et refroidis dans un bain de glace. 558mg (1.5 eq) d'hydrure de sodium (60%) sont ajoutés lentement et le milieu est agité à 0°C pendant 20 minutes. Puis le chlorure de tosyle (2.11g, 1,2 eq) est ajouté, et le milieu est agité à TA pendant 5 heures. Le solvant est évaporé. Le résidu obtenu est suspendu dans un minimum d'éthers de pétrole et filtré. Le précipité est lavé deux fois avec une solution d'hydroxyde de sodium 2M, puis séché sous vide pendant une nuit.

**RMN ¹H** (400 MHz, DMSO-d6) δ (ppm) 8.52 (d, *J* = 1.9, 1H), 8.22 (s, 1H), 8.01 (m, 3H), 7.44 (d, *J* = 8.2, 2H), 2.51 (s, 3H).
**Rendement** : 97% ; **HPLC** : 99% ; **MS [M+1]** : 476.9-478.9

### 2^{ème} étape, synthèse générale des 3-5-bis-aryl-1H-pyrrolo[2,3-b]pyridines

Méthode 1 : Dans un schlenck sous argon, le 5-bromo-3-iodo-1-(toluène-4-sulfonyl)-1*H-*pyrrolo [2,3-b] pyridine (100 mg), le premier acide boronique à coupler (1 eq) et K2CO3 (3 eq) sont, suspendus dans un mélange dioxane/eau (9/1, 3 mL), Le mélange est dégazé sous Argon pendant 20 minutes. Pd(dppf)Cl₂ (2%) est ajouté et le mélange est agité pendant 5 heures à reflux. Après contrôle de l'achèvement de la réaction de couplage par analyse LC/MS, le second acide boronique (1,2 eq) et K2CO3 (3 eq) sont ajoutés au milieu qui est dégazé à nouveau sous argon pendant 20 minutes. Pd(dppf)Cl₂ (2%) est alors ajouté et le mélange est agité à reflux pendant la nuit. Les solvants sont évaporés, le résidu est repris dans l'acétate d'éthyle. La phase organique est, lavée avec une solution aqueuse saturée en NaHCO3, puis une solution aqueuse saturée en NaCl, séchée sur Na2SO4 anhydre, filtrée et évaporée. Le milieu est repris dans un mélange de tétrahydrofurane/ méthanol (1/1, 6 mL) et agité pendant 3 heures à TA avec du carbonate de césium (3 eq). Les solvants sont évaporés, le résidu est repris dans l'acétate d'éthyle, lavé avec une solution aqueuse saturée en NaHCO3, puis une solution aqueuse saturée en NaCl, séché sur Na2SO4 anhydre, filtré et évaporé à sec, puis purifié sur colonne de silice phase inverse (eau/acétonitrile) avec 1% d'acide trifluoroacétique. Le composé est isolé après neutralisation avec NaHCO3.

Méthode 2 : Le 5-bromo-3-iodo-1-(toluène-4-sulfonyl)-1*H*-pyrrolo [2,3-b] pyridine (III) (100 mg),le premier acide boronique à coupler (IV) (1 eq) et le carbonate de potassium (3 eq) sont suspendus dans un mélange dioxane/eau (3/1, 3 mL), et le mélange est dégazé sous argon pendant 20 minutes. Pd(dppf)Cl₂ (2%) est ajouté et le mélange est agité à 80°C sous irradiation micro-ondes pendant 1 à 3 fois 20 minutes jusqu'à consommation totale du réactif de départ. Le milieu réactionnel est lavé par une solution aqueuse saturée en NaHCO3 et extrait à l'acétate d'éthyle. Les solvants sont évaporés, le résidu est repris dans l'acétate d'éthyle, lavé avec une solution aqueuse saturée en NaHCO3, séché sur Na2SO4 anhydre, filtré et évaporé à sec, puis purifié sur colonne de silice. Le composé est alors dissout dans un mélange dioxane/eau (3/1, 4 mL), en présence du second acide boronique ou son ester de pinacol (VI) (1,2 eq) et K2CO3 (3 eq). Le milieu est dégazé à nouveau sous argon pendant 20 minutes, Pd(dppf)Cl₂ (0.025 eq) est alors ajouté et le mélange est agité à 120°C pendant 45 minutes sous irradiation micro-ondes. 500 µL de soude 1M (3 eq) sont ajouté au milieu réactionnel et agité à 100°C pendant 45 minutes sous irradiation micro-ondes. Le milieu réactionnel est lavé avec une solution aqueuse saturée en NaHCO3, extrait à l'acétate d'éthyle, séché sur Na2SO4 anhydre, filtré et évaporé à sec, puis purifié sur colonne de silice phase normale (dichlorométhane/méthanol-ammoniaque).

| | |
|---|---|
| **4-Fluoro-3-(5-thiophen-3-yl-*1H*-pyrrolo[2,3-b]pyridin-3-yl)-benzaldéhyde** | **Réactifs** : Premièrement l'acide 2-Fluoro-5-formylbenzèneboronique puis l'acide 3-thiénylboronique selon la méthode 2 |
| 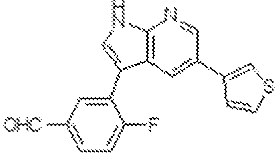 | |
| | **Rendement** : 75% ; **HPLC :** 97% ; **MS [M+1]** : 323.1 |
| **3-(3-Nitro-phényl)-5-phényl-*1H*-pyrrolo[2,3-b]pyridine** | **Réactifs** : Premièrement l'acide 3-nitrophénylboronique puis l'acide phénylboronique selon la méthode 2 |
| 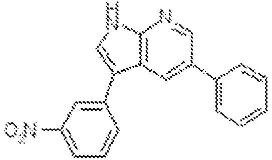 | |
| | **Rendement** : 98% ; **HPLC** : 94 % ; **MS [M+1]** : 316.1 |
| **3-(2-Fluoro-5-nitro-phényl)-5-phényl-*1H-*pyrrolo[2,3-b]pyridine** | **Réactifs** : Premièrement l'acide 2-(2-Fluoro-5-nitro-phényl)-4,4,5,5-tétraméthyl-[1,3,2]dioxaborolane puis l'acide phénylboronique selon la méthode 2 |
| 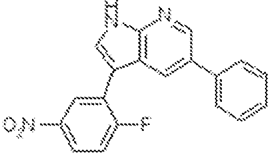 | |
| | **Rendement** : 52% ; **HPLC** : 96% ; **MS [M+1]** : 334.1 |

### Synthèse des amines

### Méthode 1 : Synthèse des amines par réduction des composés nitrés correspondants.

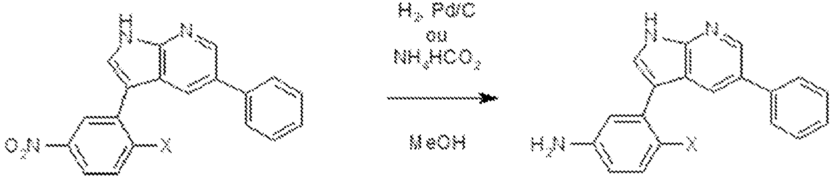

Protocole 1 : Le dérivé nitro est placé dans 200 mL de méthanol dans un autoclave. 10% massique de palladium sur charbon 10% est ajouté sous argon. L'autoclave est fermé, purgé en Argon, et placé sous 30 bars d'hydrogène. Le milieu est agité à température ambiante pendant une nuit. L'autoclave est ensuite vidé, purgé en Argon. Le milieu est filtré sur célite puis lavé au méthanol. Le filtrat est évaporé, repris en acétate d'éthyle, lavé deux fois avec une solution aqueuse saturée en NaHCO3, une fois avec une solution aqueuse saturée en NaCl, séché sur Na2SO4, filtré sur coton et évaporé à sec pour donner le dérivé amine désiré.

Protocole 2 : 100 mg (0.3 mmol) de dérivé nitro et 189 mg (10 eq) de formiate d'ammonium sont mis en suspension dans 3 mL de méthanol sous argon. Une suspension de 15 mg de palladium sur charbon dans 1 mL de méthanol est alors ajoutée et le tout est agité pendant 30 minutes à 130°C sous irradiations micro-ondes. Le milieu est filtré sur célite puis lavé au méthanol. Le filtrat est évaporé à sec puis purifié sur colonne de silice phase normale (éluant : dichlorométhane/méthanol 97/3).

| | |
|---|---|
| **3-(5-Phényl-*1H*-pyrrolo[2,3-b]pyridin-3-yl)-phénylamine** | **Réactif** : 3-(3-Nitro-phényl)-5-phényl-*1H*-pyrrolo[2,3-b]pyridine selon le protocole 1 |
| 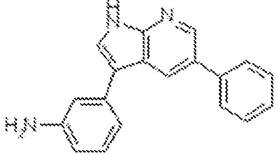 | |
| | **Rendement** : 72% ; **HPLC** : 85% ; **MS [M+1]** : 286.2 |
| **4-Fluoro-3-(5-phényl-*1H*-pyrrolo[2,3-b]pyridin-3-yl)-phénylamine** | **Réactif** : 3-(2-Fluoro-5-nitro-phényl)-5-phényl-*1H*-pyrrolo[2,3-b]pyridine selon le protocole 2 |
| 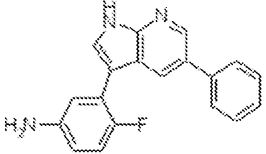 | |
| | **Rendement** : 70% ; **HPLC** : 98% ; **MS [M+1]** : 304.1 |

### Synthèse des différents acides carboxyliques non commerciaux :

L'acide 1-(4-fluoro-phényl)-6-méthyl-2-oxo-1,2-dihydro-pyridine-3-carboxylique a été obtenu conformément à la procédure décrite [Flynn et al. Organic Process Research & Development (2014), 18(4), 501-510] (**HPLC** : 97% ; **MS [M+1]** : 248.2).

L'acide 1-(4-fluoro-phénylcarbamoyl)-cyclopropane carboxylique a été obtenu conformément à la procédure décrite [Zhan et al. Medicinal Chemistry Letters (2014), 5(6), 673-678] (**HPLC** : 100% ; **MS [M+1]** : 224.1)

### Synthèse générale des composés amides à partir des composés amino-7-azaindoles et d'acides carboxyliques.

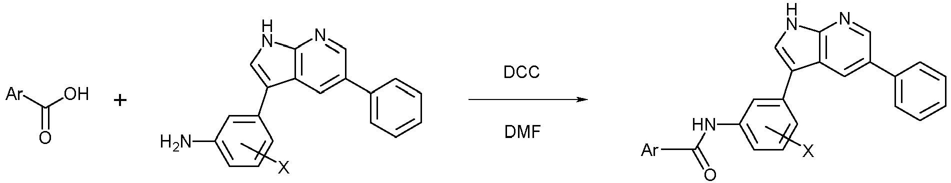

L'acide carboxylique (1eq) est dissout dans du DMF anhydre et placé dans un schlenck séché, sous atmosphère d'Argon. La N,N'-dicyclohexylcarbodiimide (1eq), puis le dérivé amino-7-azaindole (1 eq) sont ajoutés. Le milieu est alors agité à 70°C pendant une nuit. Le solvant est évaporé. Le résidu obtenu est alors dissout dans de l'acétate d'éthyle, lavé deux fois avec une solution aqueuse saturée en NaHCO3, une fois avec une solution aqueuse saturée en NaCl, séché sur sulfate de sodium anhydre, filtré sur coton, évaporé à sec, et purifié sur colonne de silice phase inverse (eau/acétonitrile) avec 1% d'acide trifluoroacétique pour donner le dérivé amide désiré.

| | |
|---|---|
| **1-(4-Fluoro-phényl)-6-méthyl-2-oxo-1,2-dihydro-pyridine-3-carboxylique acide [3-(5-phényl-*1H*-pyrrolo[2,3-b]pyridin-3-yl)-phényl]-amide (Exemple 4)** | **Réactifs** : 3-(5-Phényl-*1H*-pyrrolo[2,3-b]pyridin-3-yl)-phénylamine et acide 1-(4-Fluoro-phényl)-6-méthyl-2-oxo-1,2-dihydro-pyridine-3-carboxylique |
| 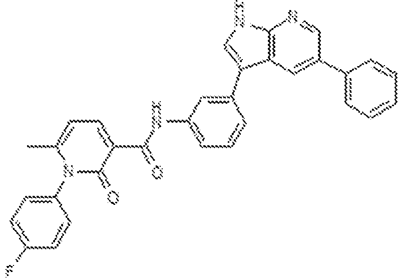 | **Rendement** : 53% ; **HPLC** : 98% ; **t_{R}** : 5.17 min (Méthode Y) ; **MS [M+1]** : 515.2 |
| **1-(4-Fluoro-phényl)-6-méthyl-2-oxo-1,2-dihydro-pyridine-3-carboxylique acide [4-fluoro-3-(5-phényl-*1H*-pyrrolo[2,3-b]pyridin-3-yl)-phényl]-amide (Exemple 15)** | **Réactifs** : 4-Fluoro-3-(5-phényl-*1H-*pyrrolo[2,3-b]pyridin-3-yl)-phénylamine et acide 1-(4-Fluorophényl)-6-méthyl-2-oxo-1,2-dihydro-pyridine-3-carboxylique |
| 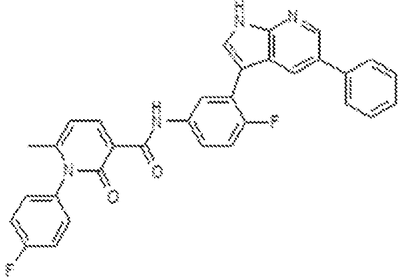 | **Rendement** : 38% ; **HPLC** : 97% ; **t_{R}** : 5.34 min (Méthode Y) ; **MS [M+1]** : 533.2 |
| **Cyclopropane-1,1-dicarboxylique acide (4-fluoro-phényl)-amide [3-(5-phényl-*1H-*pyrrolo[2,3-b]pyridin-3-yl)-phényl]-amide (Exemple 5)** | **Réactifs** : 3-(5-Phényl-*1H*-pyrrolo[2,3-b]pyridin-3-yl)-phénylamine et acide 1-(4-Fluoro-phénylcarbamoyl)-cyclopropanecarboxylique |
| 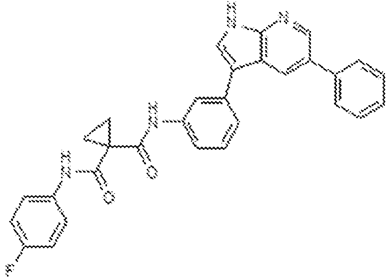 | **Rendement** : 31% ; **HPLC** : 98% ; **t_{R}** : 5.10 min (Méthode Y) ; **MS [M+1]** : 491.2 |

### Amination réductrice à partir d'amino-azaindoles

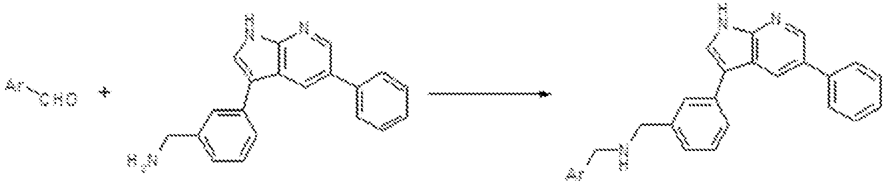

Le dérivé aldéhyde (1 eq) et le dérivé amine (1 eq) sont placés dans un ballon et dissout dans un mélange méthanol / acide acétique (9/1). Le mélange est agité 3 heures à température ambiante puis le cyanoborohydrure de sodium (2 eq) est ajouté et le milieu est agité à température ambiante pour la nuit. Le solvant est évaporé, le résidu est repris en acétate d'éthyle, lavé deux fois avec une solution aqueuse saturée en NaHCO3, une fois avec une solution aqueuse saturée en NaCl, séché sur Na2SO4anhydre, filtré sur coton et évaporé à sec. Le résidu obtenu est purifié sur colonne de silice avec un gradient de 100% dichlorométhane à 90/10 dichlorométhane/méthanol-ammoniacal.

### Amination réductrice à partir des formyl-azaindoles

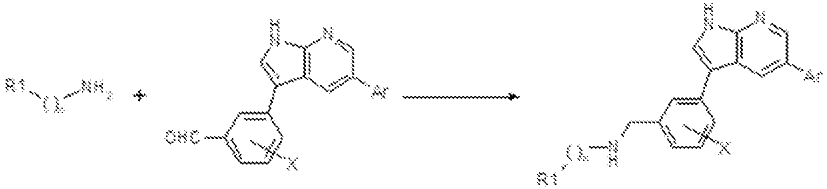

Le dérivé aldéhyde (1 eq) et le dérivé amine (1 eq) sont dissous dans un mélange méthanol / acide acétique (9/1). Le mélange est agité 3 heures à température ambiante puis le cyanoborohydrure de sodium (2 eq) est ajouté et le milieu est agité à température ambiante pour la nuit. Le solvant est évaporé, le résidu est repris en acétate d'éthyle, lavé deux fois avec une solution aqueuse saturée en NaHCO3, une fois avec une solution aqueuse saturée en NaCl, séché sur Na2SO4 anhydre, filtré sur coton et évaporé à sec. Le résidu obtenu est purifié sur colonne de silice avec un gradient de 100% dichlorométhane à 90/10 dichlorométhane/méthanol-ammoniacal.

| | |
|---|---|
| **[4-Fluoro-3-(5-thiophen-2-yl-*1H-*pyrrolo[2,3-b]pyridin-3-yl)-benzyl]-(1H-imidazol-2-ylméthyl)-amine (Exemple 6)** | **Réactifs** : 4-Fluoro-3-(5-thiophen-3-yl-*1H*-pyrrolo[2,3-b]pyridin-3-yl)-benzaldéhyde et (*1H*-Imidazol-2-yl)-méthylamine |
| 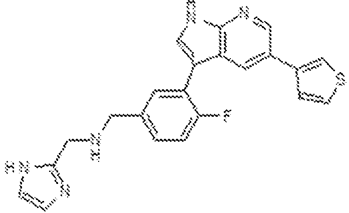 | **Rendement** : 57% ; **HPLC** : 94% ; **t_{R}** : 2.80 min (Méthode Y) ; **MS [M+1]** : 404.1 |

### Exemple 2. TESTS BIOLOGIQUES

Les composés de l'invention ont été évalués pour leur activité antivirale in vitro, en testant l'effet inhibiteur des molécules sur la réplication de virus complets infectieux. Les systèmes cellulaires choisis (cellules HeLa, A549, A549-ACE2) sont pertinents pour les modèles infectieux envisagés et ont été validés pour l'expression de la molécule Axl en cytométrie de flux.

### Modèles infectieux utilisés :

| **Virus** | **Modèle** | **Souche** | **Cellules** |
|---|---|---|---|
| ZIKV | Rapporteur mCherry | BeH-8 | HeLa |
| DENV | Rapporteur luciférase | DENV2 | HeLa |
| WNV | Rapporteur luciférase | Kunjin (KUNV) | HeLa |
| CHIKV | Rapporteur luciférase | LR OPY-1 | HeLa |
| MAYV | Rapporteur luciférase | TRVL4675 | HeLa |
| SARS-CoV2 | Isolat viral | BetaCoV/France/IDF0371/202 | VeroE6 |

### 1- Activité antivirale contre le virus Zika

Les essais sont réalisés en préincubant 2.5 x10⁴ cellules de la lignée HeLa (carcinome cervical ; ATCC# CCL-2) cultivées en plaque 96 puits avec des concentrations croissantes de composés pendant 1h à 37°C. Les cellules sont cultivées en atmosphère 5% CO2 dans un milieu Dulbecco's modified Eagle's medium (DMEM) comprenant 10% de sérum de veau et 1% de pénicilline/streptomycine. Les cellules sont ensuite exposées à la souche ZIKV BeH8 (MOI = 0.1) pendant 1h en présence du composé.

L'inoculum viral est ensuite éliminé et les cellules sont maintenues en présence du composé pendant 24 h. L'infection des cellules est déterminée par quantification des ARN viraux détectés par qRT-PCR à l'aide du kit Luna Universal One-Step RT-PCR kit. Les valeurs (DCT) sont normalisées par rapport à la quantification des mRNA de la GAPDH. Les valeurs représentées sont les moyennes de triplicats + écart type.

Les conditions contrôle ont été réalisées en incubant les cellules en présence de DMSO (0), solvant utilisé pour la solubilisation des composés testés. Les volumes de DMSO utilisés dans ces conditions correspondent aux volumes apportés par les composés dans les conditions test.

Les résultats obtenus pour le composé 4 sont illustrés en Figure 1. L'IC₅₀ du composé 4 a été déterminée à l'aide du logiciel Graphpad Prism

Dans ces conditions expérimentales, le composé 4 utilisé à une concentration supérieure ou égale à 100 nM prévient l'infection des cellules HeLa par la Souche ZIKV BeH-8.

### 2- Activité antivirale contre le virus WNV souche Kuniin

Les essais sont réalisés en préincubant 4x10⁴ cellules de la lignée HeLa (ATCC# CCL-2) cultivées en plaque 96 puits avec des concentrations croissantes de composés pendant 1h. Les cellules sont cultivées en atmosphère 5% CO2 dans un milieu Dulbecco's modified Eagle's medium (DMEM) comprenant 10% de sérum de veau et 1% de pénicilline/streptomycine. Les cellules sont ensuite exposées au virus WNV, souche Kunjin exprimant un gène nanoluciférase en amont de la séquence codant pour la protéine de capside, en présence des composés, pendant 1 h. L'inoculum viral est ensuite éliminé et les cellules sont maintenues en présence des inhibiteurs pendant 24 h.

Les cellules sont lysées à l'aide du réactif Passive lysis Buffer (Promega). L'infection virale est détectée par quantification de l'activité nanoluciferase dans le lysat des cellules en présence du réactif Genofax A (Yelen) et à l'aide d'un fluorimètre Infinite F200PRO (Tecan). Les valeurs sont normalisées par rapport à la quantité de protéines totales contenues dans le lysat cellulaire et déterminées à l'aide du kit BCA Protein Assay (Pierce) par mesure de l'absorbance à 562 nm. Les valeurs représentées sont les moyennes de triplicates + écart type. Les conditions contrôle sont identiques à celles décrites précédemment.

Les résultats obtenus pour les composés 4 et 15 sont illustrés en Figure 2.

Dans ces conditions expérimentales, les composés 4 et 15 utilisés à une concentration supérieure ou égale à 500 nM préviennent l'infection des cellules HeLa par le WNV souche Kunjin.

### 3- Activité antivirale contre le virus Chikungunya

Les essais sont réalisés en préincubant 4x10⁴ cellules de la lignée HeLa (ATCC# CCL-2) cultivées en plaque 96 puits avec des concentrations croissantes de composés pendant 1h. Les cellules sont cultivées en atmosphère 5% CO2 dans un milieu Dulbecco's modified Eagle's medium (DMEM) comprenant 10% de sérum de veau et 1% de pénicilline/streptomycine. Les cellules sont ensuite exposées au virus CHIKV, souche LR-OPY1 exprimant soit un gène luciférase, soit une séquence codant la GFP en amont de la séquence codant pour la protéine nsP3. Les cellules sont incubées pendant 1 h .

L'inoculum viral est ensuite éliminé et les cellules sont maintenues en présence des inhibiteurs pendant 24 h. L'infection virale est mesurée après lyse des cellules à l'aide du réactif Passive lysis Buffer (Promega), soit par quantification directe de la fluorescence de la GFP soit par quantification de l'activité luciferase dans le lysat des cellules réalisée en présence du réactif Genofax A (Yelen) et à l'aide d'un fluorimètre Infinite F200PRO (Tecan). Les valeurs sont normalisées par rapport à la quantité de protéines totales contenues dans le lysat cellulaire et déterminées à l'aide du kit BCA Protein Assay (Pierce) par mesure de l'absorbance à 562 nm. Dans les deux protocoles, les valeurs sont normalisées par rapport à la quantité de protéines totales contenues dans le lysat cellulaire et déterminées à l'aide du kit BCA Protein Assay (Pierce). Les valeurs représentées sont les moyennes de triplicates + écart type. Les conditions contrôle sont identiques à celles décrites précédemment.

Les résultats obtenus pour le composé 4 sont illustrés en Figure 3.

Dans ces conditions expérimentales, le composé 4 utilisé à une concentration supérieure ou égale à 100 nM prévient l'infection des cellules HeLa par le virus Chikungunya.

### 4- Activité antivirale contre le virus Mayaro

Les essais sont réalisés en préincubant 4x10⁴ cellules de la lignée HeLa (ATCC# CCL-2) cultivées en plaque 96 puits avec des concentrations croissantes de composés pendant 1h. Les cellules sont cultivées en atmosphère 5% CO2 dans un milieu Dulbecco's modified Eagle's medium (DMEM) comprenant 10% de sérum de veau et 1% de pénicilline/streptomycine. Les cellules sont ensuite exposées au virus MAYV, souche TRVL4576 exprimant un gène luciférase en amont de la séquence codant pour la protéine nsP3. Les cellules sont incubées pendant 1 h. L'inoculum viral est ensuite éliminé et les cellules sont maintenues en présence des inhibiteurs pendant 24 h.

Les cellules sont lysées à l'aide du réactif Passive lysis Buffer (Promega). L'infection virale est détectée par quantification de l'activité luciférase dans le lysat des cellules réalisée en présence du réactif Genofax A (Yelen) et à l'aide d'un fluorimètre Infinite F200PRO (Tecan). Les valeurs sont normalisées par rapport à la quantité de protéines totales contenues dans le lysat cellulaire et déterminées à l'aide du kit BCA Protein Assay (Pierce) par mesure de l'absorbance à 562 nm. Dans les deux protocoles, les valeurs sont normalisées par rapport à la quantité de protéines totales contenues dans le lysat cellulaire et déterminées à l'aide du kit BCA Protein Assay (Pierce). Les valeurs représentées sont les moyennes de triplicats + écart type. Les conditions contrôle sont identiques à celles décrites précédemment.

Les résultats obtenus pour le composé 4 sont illustrés en Figure 4.

Pour chacun des modèles infectieux testés, les IC₅₀ des composés actifs ont été déterminées à l'aide du logiciel Graphpad Prism.

| **Composé** | | | | |
|---|---|---|---|---|
| | **CHIKV** | **MAYV** | **KUNV** | **ZIKV** |
| **4** | 0,243 µM | 0,3127 µM | 1,321 µM | 0,290 µM |
| **15** | | | 0,934 µM | |
| **R428/Bemcentinib (référence)** | 0,239 µM | ND | ND | 0,285 µM |

### 6- Activité antivirale contre le coronavirus SARS-CoV2

L'activité inhibitrice des composés de l'invention sur l'infection par le SARS-CoV2 a été évaluée in vitro par infection la lignée de singe vert VeroE6 (épithélium immortalisé de rein, ATCC#CRL-1586).

### Évaluation des activités antivirales en cellules VeroE6

Les tests d'infection sont réalisés en préincubant 4x10⁴ cellules VeroE6, cultivées en plaque 96 puits, avec les concentrations indiquées de composés pendant 1h. Les cellules sont cultivées en atmosphère 5% CO2 dans un milieu Dulbecco's modified Eagle's medium (DMEM) comprenant 2% de sérum de veau et 1% de pénicilline/streptomycine. Les cellules ont ensuite été exposées au virus SARS-CoV2, souche BetaCoV/France/IDF0371/2020 (MOI = 0.01) pendant 2h avant élimination de l'inoculum, lavage et relise en culture des cellules. 24h après le challenge viral, L'infection est quantifiée par amplification des ARN viraux par qRT-PCR à l'aide du kit Luna Universal One-Step RT-PCR kit. Les valeurs (ΔCT) sont normalisées par rapport à la quantification des mRNA de la GAPDH. Les valeurs représentées sont les moyennes de triplicats + écart type. Les conditions contrôle ont été réalisées en incubant les cellules en présence de DMSO (0), solvant utilisé pour la solubilisation des composés testés. Les volumes de DMSO utilisés dans ces conditions correspondent aux volumes apportés par les composés dans les conditions test.

Un traitement parallèle, dans les mêmes conditions expérimentales, a été effectué avec le R428/Bemcentinin, inhibiteurs d'Axl en phase d'essai clinique développé par la société Bergenbio.

Les résultats obtenus pour le composé 5 sont illustrés en Figure 5.

Dans ces conditions expérimentales, le composé 5 réduit l'infection des cellules par le virus SARS-CoV2.

## Revendications

1. Composé de formule (I) : 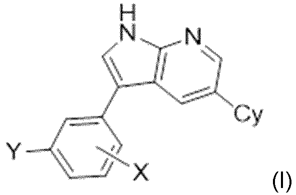 dans laquelle
X représente un atome d'hydrogène ou un atome d'halogène,
Y est choisi parmi -CH₂OH, -CH=NOH, -CH₂NH₂, un aryle, un hétéroaryle, et -L-(CH₂)p-W,
- L représente -C(O)NH-, -CH₂NH-, -CH=N-, -NHC(O)-, ou -CH₂N=CH-,
- p est un entier de 0 à 2,
- quand L représente -CH₂NH-, -CH=N-, ou -CH₂N=CH-, W est choisi parmi :
• un alkyle en C₁-C₆,
• un aryle en C₆-C₁₀, éventuellement substitué par 1 ou 2 groupes indépendamment choisis parmi
∘ un atome d'halogène,
∘ un hydroxyle,
∘ un alcoxyle en C₁-C₆,
∘ un alkyle en C₁-C₆ dans lequel 1 ou plusieurs atomes d'hydrogène est éventuellement remplacé par un atome de fluor, et
∘ un hétéroaryle à 5-10 chaînons comprenant de 1 à 3 hétéroatomes indépendamment choisis parmi N, O et S ;
• un hétéroaryle à 5-10 chaînons contenant de 1 à 3 hétéroatomes indépendamment choisis parmi N, O et S, ledit hétéroaryle étant éventuellement substitué par 1 à 4 groupes indépendamment choisis parmi
∘ un atome d'halogène,
∘ un groupe oxo,
∘ un hydroxyle,
∘ un alcoxyle en C₁-C₆,
∘ un alkyle en C₁-C₆, dans lequel 1 ou plusieurs atomes d'hydrogène est éventuellement remplacé par un atome de fluor, et
∘ un aryle en C₆-C₁₀ éventuellement substitué par un atome d'halogène et/ou un alkyle en C₁-C₆, ou un térazole substitué par un alkyle en C₁-C₆ ;
- quand L représente -C(O)NH- ou -NHC(O)-, W est choisi parmi:
• un aryle en C₆-C₁₀,éventuellement substitué par un atome d'halogène, un hydroxyle, un alkyle en C₁-C₆, ou un alcoxyle en C₁-C₆,
• un hétéroaryle à 5-10 chaînons comprenant de 1 à 2 hétéroatomes indépendamment choisis parmi N, O et S ledit hétéroaryle étant éventuellement substitué par 1 à 4 substituants indépendamment choisis parmi
∘ un atome d'halogène,
∘ un hydroxyle,
∘ un groupe oxo,
∘ un alcoxyle en C₁-C₆,
∘ un alkyle en C₁-C₆ dans lequel 1 ou plusieurs atomes d'hydrogène est éventuellement remplacé par un atome de fluor, et
∘ un aryle en C₆-C₁₀ éventuellement substitué par un atome d'halogène et/ou un alkyle en C₁-C₆; et
• un cycloakyle en C₃-C₆ éventuellement substitué par un groupe C(O)NHR ou NHC(O)R dans lequel R représente un aryle en C₆-C₁₀ éventuellement substitué par un atome d'halogène et/ou un alkyle en C₁-C₆;
Cy représente un phényle ou un hétéroaryle à 5-10 chaînons contenant de 1 à 3 hétéroatomes indépendamment choisis parmi N, O et S, ledit hétéroaryle étant éventuellement substitué par un groupe oxo,
ou un sel pharmaceutiquement acceptable de celui-ci ou un de leurs mélanges, pour son utilisation dans la prévention et/ou le traitement des infections virales associées au récepteur AXL choisies parmi le groupe constitué des infections virales dues aux Flavivirus, tels que les virus de la dengue, Zika, West Nile, Kunjin ; aux Alphavirus, tels que les virus Chikungunya, Mayaro, Semliki Forest, Sindbis, le virus de l'encéphalite équine de l'Est, le virus de l'encéphalite équine Occidental, le virus de l'encéphalite équine du Venezuela ; aux Filovirus tels que le virus Ebola, le virus de la fièvre de Marburg ; aux arénavirus tels que le virus de la fièvre de Lassa, le virus Junin, le virus Amapari ; aux picornavirus tels que le virus de la stomatite vésiculeuse ; aux paramyxovirus, tels que le virus influenza ; ou aux coronavirus tels que le SARS-COV2.

2. Composé pour utilisation selon la revendication 1, de formule (la): 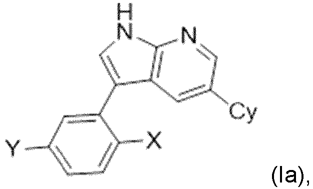 dans laquelle X, Y et Cy sont tels que définis dans la revendication 1.

3. Composé pour utilisation selon la revendication 1 ou 2, **caractérisé en ce que** Cy représente un groupe phényle, thiophène, furane, pyridine ou pyrimidine.

4. Composé pour utilisation selon l'une quelconque des revendications 1 à 3, **caractérisé en ce que** X représente un halogène.

5. Composé pour utilisation selon l'une quelconque des revendications 1 à 4, **caractérisé en ce que** Y représente L-(CH₂)ₚ-W, L représentant CH₂NH, p étant un entier de 0 à 2, et W étant choisi parmi :
- un phényle éventuellement substitué par 1 ou 2 groupes indépendamment choisis parmi un atome de brome, un atome de fluor, un groupe hydroxyle, méthoxy, un trifluorométhyle, ou triazole, et
- un hétéroaryle choisi parmi un furane, pyrrole, imidazole, pyrazole, thiophene, pyridine, pyrimidine, benzofurane, benzodioxole, indole et benzimidazole, ledit hétéroaryle étant éventuellement substitué par 1 à 4 groupes indépendamment choisis parmi un atome d'halogène, un hydroxyle, un groupe oxo, un méthoxy, un méthyle, un trifluorométhyle, un phényle et un fluorophényle.

6. Composé pour utilisation selon la revendication 5, **caractérisé en ce que** W est un imidazole.

7. Composé pour utilisation selon l'une quelconque des revendications 1 à 4, **caractérisé en ce que** Y représente NHC(O)-W, W étant choisi parmi :
- la 2(1H)-pyridinone éventuellement substituée par 1 à 2 substituants indépendamment choisis parmi un aryle en C₆-C₁₀ éventuellement substitué par un atome d'halogène, et un méthyle, et
- un cycloakyle en C₃-C₆ éventuellement substitué par un groupe -C(O)NHR ou -NHC(O)R dans lequel R représente un aryle en C₆-C₁₀, éventuellement substitué par un atome d'halogène.

8. Composé pour utilisation selon la revendication 1, choisi parmi :

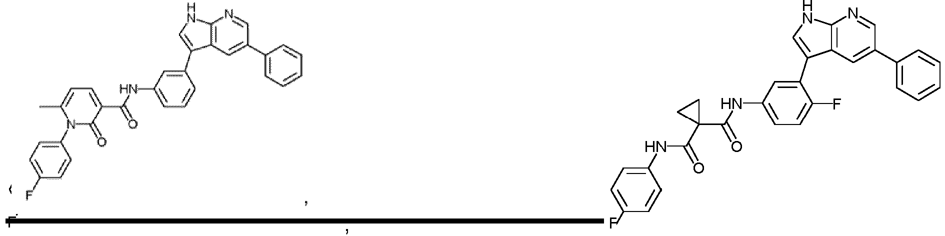

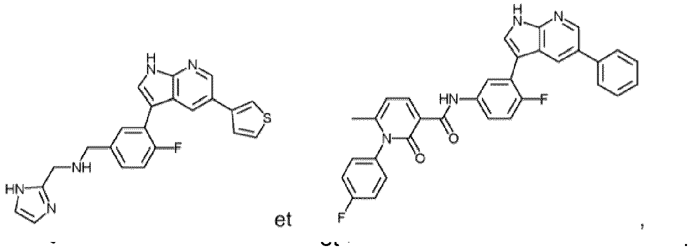

9. Composition pharmaceutique comprenant un composé selon l'une quelconque des revendications 1 à 8 ou un sel pharmaceutiquement acceptable de celui-ci en tant que principe actif, et un excipient pharmaceutiquement acceptable pour son utilisation dans la prévention et/ou le traitement des infections virales associées au récepteur AXL choisies parmi le groupe constitué des infections virales dues aux Flavivirus, tels que les virus de la dengue, Zika, West Nile, Kunjin ; aux Alphavirus, tels que les virus Chikungunya, Mayaro, Semliki Forest, Sindbis, le virus de l'encéphalite équine de l'Est, le virus de l'encéphalite équine Occidental, le virus de l'encéphalite équine du Venezuela ; aux Filovirus tels que le virus Ebola, le virus de la fièvre de Marburg ; aux arénavirus tels que le virus de la fièvre de Lassa, le virus Junin, le virus Amapari ; aux picornavirus tels que le virus de la stomatite vésiculeuse ; aux paramyxovirus, tels que le virus influenza ; ou aux coronavirus tels que le SARS-COV2.

10. Composition pharmaceutique pour utilisation selon la revendication 9, comprenant en outre un agent thérapeutique supplémentaire choisi parmi un agent antiviral, un agent anti-inflammatoire, un agent immunomodulateur ou immunosuppresseur, un agent pour le traitement de troubles d'immunodéficience et un agent pour le traitement de la douleur.

## Patentansprüche

1. Verbindung der Formel (I): 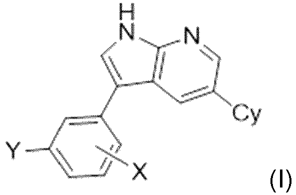 worin
**X** ein Wasserstoffatom oder ein Halogenatom darstellt,
**Y** ausgewählt ist aus -CH₂OH, -CH=NOH, -CH₂NH₂, einem Aryl, einem Heteroaryl und -L-(CH₂)ₚ-W,
- **L** steht für -C(O)NH-, -CH₂NH-, -CH=N-, -NHC(O)- oder -CH₂N=CH-,
- **p** ist eine ganze Zahl von 0 bis 2,
- wenn **L** für -CH₂NH-, -CH=N- oder -CH₂N=CH- steht, ist **W** ausgewählt aus:
• einem C₁-C₆-Alkyl,
• einem C₆-C₁₀-Aryl, optional substituiert durch 1 oder 2 Gruppen, die jeweils unabhängig voneinander ausgewählt sind aus:
∘ einem Halogenatom,
∘ einer Hydroxygruppe,
∘ einer C₁-C₆-Alkoxygruppe,
∘ einer C₁-C₆-Alkylgruppe, wobei ein oder mehrere Wasserstoffatome optional durch Fluoratome ersetzt sind, und
∘ einem 5- bis 10-gliedrigen Heteroaryl, das 1 bis 3 Heteroatome enthält, die unabhängig voneinander aus N, O und S ausgewählt sind,
• einem 5- bis 10-gliedrigen Heteroaryl, das 1 bis 3 Heteroatome enthält, die unabhängig voneinander aus N, O und S ausgewählt sind, wobei dieses Heteroaryl optional substituiert ist mit 1 bis 4 Gruppen, die unabhängig voneinander ausgewählt sind aus:
∘ einem Halogenatom,
∘ einer Oxogruppe,
∘ einer Hydroxygruppe,
∘ einer C₁-C₆-Alkoxygruppe,
∘ einer C₁-C₆-Alkylgruppe, wobei ein oder mehrere Wasserstoffatome optional durch Fluoratome ersetzt sind, und
∘ einem C₆-C₁₀-Aryl, das optional mit einem Halogenatom und/oder einer C₁-C₆-Alkylgruppe substituiert ist, oder einem Tetrazol substituiert mit einer C₁-C₆-Alkylgruppe;
- wenn **L** für -C(O)NH- oder -NHC(O)- steht, ist **W** ausgewählt aus:
• einem C₆-C₁₀-Aryl, optional substituiert mit einem Halogenatom, einer Hydroxygruppe, einer C₁-C₆-Alkylgruppe oder einer C₁-C₆-Alkoxygruppe,
• einem 5- bis 10-gliedrigen Heteroaryl mit 1 bis 2 Heteroatomen, die unabhängig voneinander aus N, O und S ausgewählt sind, wobei dieses Heteroaryl optional substituiert ist mit 1 bis 4 Substituenten, die unabhängig voneinander ausgewählt sind aus:
∘ einem Halogenatom,
∘ einer Hydroxygruppe,
∘ einer Oxogruppe,
∘ einer C₁-C₆-Alkoxygruppe,
∘ einer C₁-C₆-Alkylgruppe, wobei ein oder mehrere Wasserstoffatome optional durch Fluoratome ersetzt sind, und
∘ einem C₆-C₁₀-Aryl, das optional mit einem Halogenatom und/oder einer C₁-C₆-Alkylgruppe substituiert ist; und
• einem C₃-C₆-Cycloalkyl, optional substituiert mit einer C(O)NHR- oder einer NHC(O)R-Gruppe, wobei **R ein** C₆-C₁₀-Aryl darstellt, das optional mit einem Halogenatom und/oder einer C₁-C₆-Alkylgruppe substituiert ist;
**Cy** steht für ein Phenyl oder ein 5- bis 10-gliedriges Heteroaryl mit 1 bis 3 Heteroatomen, die unabhängig voneinander aus N, O und S ausgewählt sind, wobei dieses Heteroaryl optional mit einer Oxogruppe substituiert ist,
oder ein pharmazeutisch verträgliches Salz davon oder eine Mischung davon,
zur Verwendung bei der Vorbeugung und/oder Behandlung von Virusinfektionen, die mit dem AXL-Rezeptor assoziiert sind, ausgewählt aus der Gruppe bestehend aus Virusinfektionen durch Flaviviren, wie Denguevirus, Zika-Virus, West-Nil-Virus, Kunjin-Virus, durch Alphaviren, wie Chikungunya-Virus, Mayaro-Virus, Semliki-Forest-Virus, Sindbis-Virus, Ost-Equinenenzephalitis-Virus, West-Equinenenzephalitis-Virus, Venezuelanisches-Equinenenzephalitis-Virus, durch Filoviren wie Ebolavirus, Marburgvirus, durch Arenaviren wie Lassafiebervirus, Juninvirus, Amaparivirus, durch Picornaviren wie Vesikuläres Stomatitisvirus, durch Paramyxoviren wie Influenzavirus oder durch Coronaviren wie SARS-CoV-2.

2. Verbindung zur Verwendung gemäß Anspruch 1, von der Formel (la): 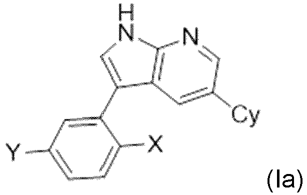 wobei X, Y und Cy wie in Anspruch 1 definiert sind.

3. Verbindung zur Verwendung gemäß Anspruch 1 oder 2, **dadurch gekennzeichnet, dass** Cy eine Phenyl-, Thiophen-, Furan-, Pyridin- oder Pyrimidin-Gruppe darstellt.

4. Verbindung zur Verwendung gemäß einem der Ansprüche 1 bis 3, **dadurch gekennzeichnet, dass** X ein Halogen darstellt.

5. Verbindung zur Verwendung gemäß einem der Ansprüche 1 bis 4, **dadurch gekennzeichnet, dass** Y L-(CH₂)ₚ-W darstellt, wobei L CH₂NH bedeutet, p eine ganze Zahl von 0 bis 2 ist und W ausgewählt ist aus:
- einem Phenyl, optional substituiert mit 1 oder 2 Gruppen, die unabhängig voneinander ausgewählt sind aus einem Bromatom, einem Fluoratom, einer Hydroxygruppe, einer Methoxygruppe, einer Trifluormethylgruppe oder einer Triazolgruppe, und
- einem Heteroaryl, ausgewählt aus Furan, Pyrrol, Imidazol, Pyrazol, Thiophen, Pyridin, Pyrimidin, Benzofuran, Benzodioxol, Indol und Benzimidazol, wobei dieses Heteroaryl optional substituiert ist mit 1 bis 4 Gruppen, die unabhängig voneinander ausgewählt sind aus einem Halogenatom, einer Hydroxygruppe, einer Oxogruppe, einer Methoxygruppe, einer Methylgruppe, einer Trifluormethylgruppe, einem Phenyl und einem Fluorphenyl.

6. Verbindung zur Verwendung gemäß Anspruch 5, **dadurch gekennzeichnet, dass** W ein Imidazol ist.

7. Verbindung zur Verwendung gemäß einem der Ansprüche 1 bis 4, **dadurch gekennzeichnet, dass** Y NHC(O)-W darstellt, wobei W ausgewählt ist aus:
- 2(1H)-Pyridon, optional substituiert mit 1 bis 2 Substituenten, die unabhängig voneinander ausgewählt sind aus einem C₆-C₁₀-Aryl, das optional mit einem Halogenatom substituiert ist, und einer Methylgruppe, und
- einem C₃-C₆-Cycloalkyl, optional substituiert mit einer -C(O)NHR- oder - NHC(O)R-Gruppe, wobei R ein C₆-C₁₀-Aryl darstellt, das optional mit einem Halogenatom substituiert ist.

8. Verbindung zur Verwendung gemäß Anspruch 1, ausgewählt aus:

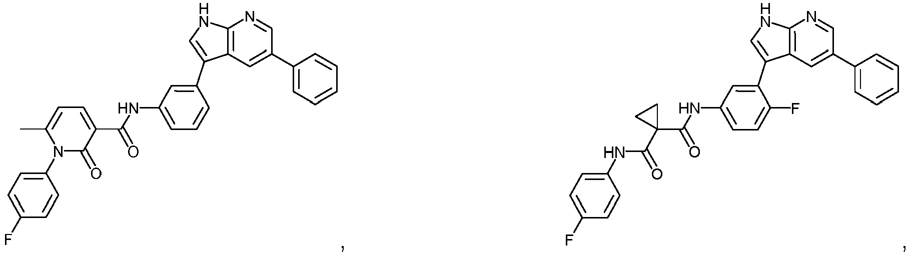

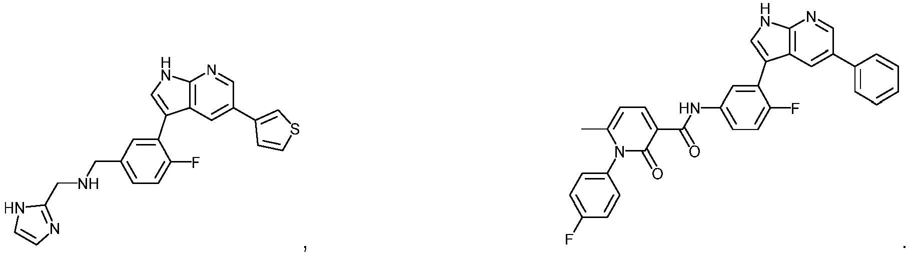

9. Pharmaceutical composition comprising a compound according to anyone of claims 1 to 8 or a pharmaceutically acceptable salt thereof as an active ingredient, and a pharmaceutically acceptable excipient for use in the prevention and/or treatment of viral infections associated with AXL receptor selected from the group consisting of viral infections due to Flaviviruses, such as dengue virus, Zika virus, West Nile virus, Kunjin virus, to Alphaviruses, such as Chikungunya virus, Mayaro virus, Semliki Forest virus, Sindbis virus, Eastern equine encephalitis virus, Western equine encephalitis virus, Venezuelan equine encephalitis virus, to Filoviruses such as Ebola virus, Marburg fever virus, to arenaviruses such as Lassa fever virus, Junin virus, Amapari virus, to picornaviruses such as vesicular stomatitis virus, to paramyxoviruses such as influenza virus, or to coronaviruses such as SARS-COV2.10. The pharmaceutical composition for use according to claim 9, further comprising an additional therapeutic agent, chosen from an antiviral agent, an anti-inflammatory agent, an immunomodulatory or immunosuppressive agent, an agent for the treatment of immunodeficiency disorders and an agent for pain treatment.

10. Pharmaceutical composition for use according to claim 9, further comprising an additional therapeutic agent selected from an antiviral agent, an anti-inflammatory agent, an immunomodulating or immunosuppressive agent, an agent for the treatment of immunodeficiency disorders and an agent for the treatment of pain.

## Claims

1. Compound of formula (I): 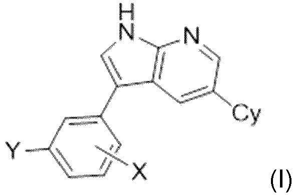 in which
X represents a hydrogen atom or a halogen atom,
Y is selected from -CH₂OH, -CH=NOH, -CH₂NH₂, an aryl, a heteroaryl, and -L-(CH₂)ₚ-W,
- L represents -C(O)NH-, -CH₂NH-, -CH=N-, -NHC(O)-, or -CH₂N=CH-,
- p is an integer from 0 to 2,
- when L represents -CH₂NH-, -CH=N-, or -CH₂N=CH-, W is selected from:
∘ a C₁-C₆ alkyl,
∘ a C₆-C₁₀ aryl, optionally substituted by 1 or 2 groups independently selected from:
▪ a halogen atom,
▪ a hydroxyl,
▪ a C₁-C₆ alkoxyl,
▪ a C₁-C₆ alkyl, wherein 1 or several hydrogen atoms is (are) optionally replaced with a fluorine atom, and
▪ a 5-10 membered heteroaryl comprising from 1 to 3 heteroatoms independently selected from N, O and S;
∘ a 5-10 membered heteroaryl containing from 1 to 3 heteroatoms independently selected from N, O and S, said heteroaryl being optionally substituted with 1 to 4 groups independently selected from:
▪ a halogen atom,
▪ an oxo group,
▪ a hydroxyl,
▪ a C₁-C₆ alkoxyl,
▪ a C₁-C₆ alkyl, wherein 1 or several hydrogen atoms is (are) optionally replaced with a fluorine atom, and
▪ a C₆-C₁₀ aryl optionally substituted with a halogen atom and/or a C₁-C₆ alkyl, or a terazole substituted with a C₁-C₆ alkyl;
- when L represents -C(O)NH- or -NHC(O)-, W is selected from:
∘ a C₆-C₁₀ aryl optionally substituted with a halogen atom, a hydroxyl, a C₁-C₆ alkyl, or a C₁-C₆ alkoxyl,
∘ a 5-10 membered heteroaryl comprising from 1 to 2 heteroatoms independently selected from N, O and S, said heteroaryl being optionally substituted with 1 to 4 substituents independently selected from:
▪ a halogen atom,
▪ a hydroxyl,
▪ an oxo group,
▪ a C₁-C₆ alkoxyl,
▪ a C₁-C₆ alkyl, wherein 1 or several hydrogen atoms is (are) optionally replaced with a fluorine atom, and
▪ a C₆-C₁₀ aryl optionally substituted with a halogen atom and/or a C₁-C₆ alkyl; and
∘ a C₃-C₆ cycloakyl optionally substituted with a C(O)NHR or NHC(O)R group wherein R represents a C₆-C₁₀ aryl optionally substituted with a halogen atom and/or a C₁-C₆ alkyl;
Cy represents a phenyl or a 5-10 membered heteroaryl containing from 1 to 3 heteroatoms independently selected from N, O and S, said heteroaryl being optionally substituted with an oxo group,
or a pharmaceutically acceptable salt thereof or a mixture thereof, for use in the prevention and/or treatment of viral infections associated with AXL receptor selected from the group consisting of viral infections due to Flaviviruses, such as dengue virus, Zika virus, West Nile virus, Kunjin virus, to Alphaviruses, such as Chikungunya virus, Mayaro virus, Semliki Forest virus, Sindbis virus, Eastern equine encephalitis virus, Western equine encephalitis virus, Venezuelan equine encephalitis virus, to Filoviruses such as Ebola virus, Marburg fever virus, to arenaviruses such as Lassa fever virus, Junin virus, Amapari virus, to picornaviruses such as vesicular stomatitis virus, to paramyxoviruses such as influenza virus, or to coronaviruses such as SARS-COV2.

2. Compound for use according to claim 1, of formula (la): 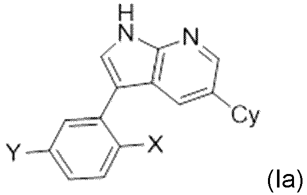 wherein X, Y and Cy are as defined in claim 1.

3. Compound for use according to claim 1 or 2, **characterized in that** Cy represents a phenyl, thiophene, furan, pyridine or pyrimidine group.

4. Compound for use according to anyone of claims 1 to 3, **characterized in that** X represents a halogen.

5. Compound for use according to anyone of claims 1 to 4, **characterized in that** Y represents L-(CH₂)ₚ-W, L representing CH₂NH, p being an integer from 0 to 2, and W being selected from:
- a phenyl optionally substituted with 1 or 2 groups independently selected from a bromine atom, a fluorine atom, a hydroxyl, methoxy, a trifluoromethyl, or triazole group, and
- a heteroaryl selected from a furan, pyrrole, imidazole, pyrazole, thiophene, pyridine, pyrimidine, benzofuran, benzodioxole, indole and benzimidazole, said heteroaryl being optionally substituted with 1 to 4 groups independently selected from a halogen atom, a hydroxyl, an oxo group, a methoxy, a methyl, a trifluoromethyl, a phenyl and a fluorophenyl.

6. Compound for use according to claim 5, **characterized in that** W is an imidazole.

7. Compound for use according to anyone of claims 1 to 4, **characterized in that** Y represents NHC(O)-W, W being selected from:
- 2(1H)-pyridinone optionally substituted with 1 to 2 substituents independently selected from C₆-C₁₀ aryl optionally substituted with a halogen atom, and a methyl, and
- a C₃-C₆ cycloakyl optionally substituted with a -C(O)NHR or -NHC(O)R group wherein R represents a C₆-C₁₀ aryl, optionally substituted with a halogen atom.

8. Compound for use according to claim 1, selected from:

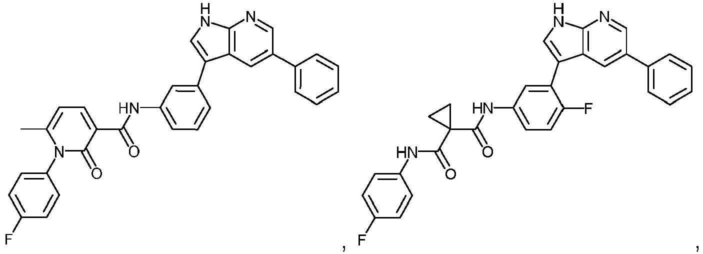

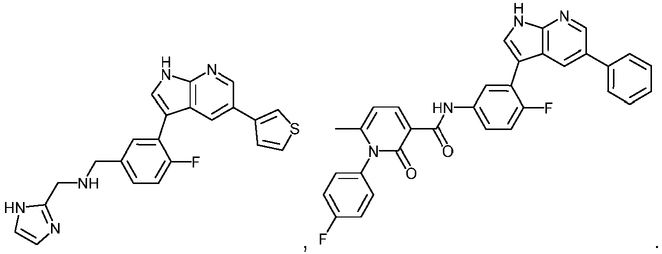

9. Pharmaceutical composition comprising a compound according to anyone of claims 1 to 8 or a pharmaceutically acceptable salt thereof as an active ingredient, and a pharmaceutically acceptable excipient for use in the prevention and/or treatment of viral infections associated with AXL receptor selected from the group consisting of viral infections due to Flaviviruses, such as dengue virus, Zika virus, West Nile virus, Kunjin virus, to Alphaviruses, such as Chikungunya virus, Mayaro virus, Semliki Forest virus, Sindbis virus, Eastern equine encephalitis virus, Western equine encephalitis virus, Venezuelan equine encephalitis virus, to Filoviruses such as Ebola virus, Marburg fever virus, to arenaviruses such as Lassa fever virus, Junin virus, Amapari virus, to picornaviruses such as vesicular stomatitis virus, to paramyxoviruses such as influenza virus, or to coronaviruses such as SARS-COV2.10. The pharmaceutical composition for use according to claim 9, further comprising an additional therapeutic agent, chosen from an antiviral agent, an anti-inflammatory agent, an immunomodulatory or immunosuppressive agent, an agent for the treatment of immunodeficiency disorders and an agent for pain treatment.

10. Pharmaceutical composition for use according to claim 9, further comprising an additional therapeutic agent selected from an antiviral agent, an anti-inflammatory agent, an immunomodulating or immunosuppressive agent, an agent for the treatment of immunodeficiency disorders and an agent for the treatment of pain.
